# EUROPÄISCHE PATENTSCHRIFT

(11) **EP 3 484 423 B1**
(45) Veröffentlichungstag und Bekanntmachung des Hinweises auf die Patenterteilung: **29.12.2021**
(21) Anmeldenummer: 17736689.5
(22) Anmeldetag: 12.07.2017
(51) Int. Cl.: A61F 7/00, A61F 7/02, A61P 17/04, A61P 43/00

(54) **VORRICHTUNG ZUR HYPERTHERMISCHEN BEHANDLUNG VON JUCKREIZ**
DEVICE FOR HYPERTHERMAL TREATMENT OF ITCHING
DISPOSITIF DE TRAITEMENT HYPERTHERMIQUE DE DÉMANGEAISONS

(30) Priorität: 12.07.2016 EP 16179093; 11.10.2016 EP 16193220
(43) Veröffentlichungstag der Anmeldung: 22.05.2019
(73) Patentinhaber: Dermapharm AG, 82031 Grünwald (DE)
(72) Erfinder: BÜNGER, Daniel, 41063 Mönchengladbach (DE)
(74) Vertreter: Hertin und Partner Rechts- und Patentanwälte PartG mbB
(86) Internationale Anmeldenummer: PCT/EP2017/067544
(87) Internationale Veröffentlichungsnummer: WO 2018/011263

(56) Entgegenhaltungen:
- EP-B1- 1 231 875
- WO-A1-2008/089327
- US-A1- 2007 049 998
- US-A1- 2011 184 502
- US-A1- 2013 172 966

## Beschreibung

Die Erfindung betrifft eine Vorrichtung zur hyperthermischen Behandlung von Juckreiz beispielsweise nach Insektenstichen, wobei während der Behandlung eine Behandlungsfläche auf eine Temperatur von bevorzugt zwischen 42 °C und 56 °C für einen Zeitraum von 2 sec bis 12 sec reguliert wird und ein hardwareimplementierter Temperaturwächter die Maximaltemperatur der Behandlungsfläche begrenzt und eine Schmelzsicherung bei Kurzschluss oder ungeregeltem Durchheizen die Vorrichtung abschaltet.

### Hintergrund und Stand der Technik

Juckreiz (Pruritus) ist eine subjektiv unangenehme, auf die Haut oder Schleimhaut bezogene Sinneswahrnehmung. Sie kann lokal begrenzt sein oder aber den ganzen Körper betreffen.

Häufig geht Juckreiz mit einem brennenden, stechenden oder kribbelnden Gefühl einher, welches die betroffene Person oftmals durch Kratzen, Scheuern, Rubbeln, Drücken, Kneten oder Reiben versucht zu lindern. Daher kommt es bei Juckreiz gehäuft zu weiteren pathologischen Erscheinung der Haut wie Kratzspuren, offene Wunden, Krustenbildung und Hautinfektionen. Die Fachwelt geht davon aus, dass Juckreiz über Schmerzrezeptoren der Haut vermittelt wird und über das vegetative Nervensystem zum Gehirn geleitet wird. Die Ursachen von Juckreiz können sehr vielfältig sein. Neben trockener Haut, fehlender Feuchtigkeitszufuhr oder Allergien kann Juckreiz auch durch äußere Einwirkungen und Hautreizungen, wie z.B. durch Stiche von Mücken oder nach Kontakt mit Nesseltieren entstehen. Juckreiz kann eine Reaktion auf chemische, mechanische oder thermische Reize sein. Es können als Folge der äußerlichen Reizung, wie z.B. durch Einwirkungen chemischer Substanzen, z.B. Histamin (Mückenstich), Apamin (Bienenstich), durch allergische Immunreaktion, durch Druck oder Reibung oder auch durch Wärme oder Sonnenbestrahlung, Quaddeln, Urtikaria und andere mit Juckreiz verbundene Hautreaktionen hervorgerufen werden. Aus medizinischer Sicht spannen die Ursachen oder Grunderkrankungen, welche zu Juckreiz führen ein breites Spektrum dermatologischer und internistischer Erkrankungen auf.

Zur medikamentösen Behandlung von den Symptomen des Juckreizes sind eine Reihe von Medikamenten oder kosmetischen Produkten bekannt. So werden ätherische Öle insbesondere umfassend Menthol, Thymol oder Campher verwandt, um kurzfristig eine Kühlung zu erzeugen. Auch Hautpflegemittel wie beispielsweise Cremen oder Lotion können durch eine Erhöhung des Feuchtigkeitsgehaltes der Haut eine schmerzlindernde Wirkung entfalten. Darüber hinaus stellen Antihistaminika hilfreiche Therapiemöglichkeiten dar, welche beispielsweise die Gabe von Dimetindenmaleat oder Mepyramin umfassen. Weitere Medikamente betreffen topische Glucocorticoide, Anästhetika, Zinksalben, Calcineurinhemmer oder Capsaicin.

Zur Behandlung von Wespen oder Bienenstichen werden weiterhin die Einstichstellen auch mit Salmiakgeist behandelt, welches jedoch nur zu einer kurzfristigen Linderung des Juckreizes führt und auch die Schwellung nur im geringen Maße zurückgehen lässt.

Aus dem Stand der Technik ist es jedoch auch bekannt durch Einbringung einer Wärmemenge in den Einstich von Insekten das Auslösen eines Juckreizes zu mindern. Eine Vorrichtung für eine lokale, thermische Behandlung insbesondere von Mückenstichen wird in der EP 1231875 B1 beschrieben. Die Vorrichtung weist eine Heizplatte mit einer Größe von ca. 0,2 cm² auf, welche auf eine Temperatur zwischen 50°C und 65°C gebracht wird, während die Heizplatte den Insektenstich kontaktiert. Durch diese hyperthermische Behandlung lässt sich der Juckreiz nachhaltig lindern. Zum einen resultiert die Wärmeapplikation in einem Abbau der thermolabilen Giftstoffe der Insekten, welche den Juckreiz auslösen. Zum anderen führt die Wärmeübertragung zu einer Überlagerung des Juckreizes durch andere temperaturabhängige Hautempfindungen. Durch derartige Behandlungen können somit weiterhin sekundäre Schädigungen der Haut, beispielsweise eine Entzündung des Insektenstiches durch Kratzen, wirksam vermieden werden. Somit wird durch die hyperthermische Behandlung auch das mit einem Insektenstich einhergehende Auftreten von Quaddeln wirksam reduziert.

Die Anwendungsmöglichkeiten einer hyperthermischen Behandlung erstrecken sich weiterhin auf Herpeserkrankungen. Aus der DE 102005002946 A1 ist eine Vorrichtung zur Behandlung von Herpeserkrankungen bekannt. Die Vorrichtung umfasst eine Heizplatte mit einer bevorzugten Größe von 20 mm², welche für eine Behandlungsdauer von vorzugsweise 10 - 15 sec auf 49 °C - 53 °C erhitzt wird. Während der Behandlungszeit kontaktiert die Heizplatte die betroffene Hautpartie der Lippen, beispielsweise die gerötete Stelle oder aber die Position an der sich bereits Bläschen gebildet haben. Die Hitzeapplikation führt zum einen zu einer Eindämmung der Vermehrung der ursächlichen Krankheitserreger durch eine neutralisierende Wirkung auf die Herpes-simplex-Viren. Zum anderen kommt es durch die kurzzeitige Wärmebehandlung zu einer Überlagerung des Juckreizes der Herpeserkrankung durch die Stimulation temperatursensitiver Nerven. Die Vorrichtung zeichnet sich somit durch eine Reduktion der Symptome der Herpeserkrankung wie z.B. Brennen, dem Auftreten von Schwellungen, einer Rötung oder eines Juckreizes aus.

Aus dem Stand der Technik aus der US 2007/0049998 A1 ist ebenfalls ein Gerät zur hyperthermischen Behandlung von Insektenstichen bekannt, dass eine Behandlungstemperatur von 50°C vorsieht. Dieses Gerät hat den Nachteil, dass bei dieser Temperatur Prozesse, die den Juckreiz lindern, noch nicht oder nicht richtig angestoßen werden. Für die hyperthermische Behandlung wesentliche Prozesse, die zur Linderung der Symptome von Insektenstichen, Herpeserkrankungen, Quallenstichen oder anderen mit Juckreiz einhergehenden Erkrankungen beitragen, werden teilweise erst in einem Temperaturbereich zwischen 50 °C und 56 °C, insbesondere zwischen 50 °C und 53 °C, aktiviert.

Die aus dem Stand der Technik bekannten Vorrichtungen zur hyperthermischen Behandlung zeichnen sich durch vielfältige Einsatzmöglichkeiten zur Linderung der Symptome von Insektenstichen, Herpeserkrankungen, Quallenstichen oder anderen mit Juckreiz einhergehenden Erkrankungen aus. Die Vorrichtungen weisen jedoch auch Nachteile auf.

So kann es bei den bekannten Vorrichtungen in Ausnahmefällen zu einer Überschreitung der gewünschten Behandlungstemperatur kommen. Im Stand der Technik ist es bekannt die Behandlungstemperatur mithilfe von Temperatursensoren zu überwachen. Durch Beschädigungen des Gerätes, beispielsweise durch das Eintreten von Feuchtigkeit, kann es jedoch zu einer Beeinträchtigung des Regelkreislaufes der Überwachungselektronik kommen. Dies ist insbesondere der Fall, wenn die Überwachung der Behandlungstemperatur in den regulären Steuerkreis eingebunden ist. In diesem Fall ist es nicht auszuschließen, dass es zu einer Überhöhung der Temperatur über die gewünschte Behandlungstemperatur hinaus kommt. In Abhängigkeit von der Kontaktposition der Heizplatte bzw. Behandlungsfläche treten hierdurch unerwünschte Nebenwirkungen auf. Selbst bei einer kurzzeitigen Temperaturerhöhung von über 65°C kann es zu nachhaltigen Schädigungen der betreffenden Hautpartien kommen. Dies ist insbesondere der Fall für empfindliche Hautpartien, wie beispielsweise die Lippen während einer Herpesbehandlung oder aber auch dünnere Hautpartien, an welchen Insektenstiche vorliegen.

Aus der US 2007/0049998 A1 ist ein Gerät zur hyperthermischen Behandlung von Hautbeschwerden bekannt, welches eine Behandlungsfläche über ein temperaturgeregeltes Heizelement auf Temperaturen von 38 °C - 67 °C für eine Dauer von mindestens 5 Sekunden, typischerweise jedoch über einen längeren Zeitraum, aufheizt und zur Sicherung gegen Überhitzen eine Schmelzsicherung verwendet. Diese Art der Absicherung gegen Überhitzen hat den Nachteil, dass bei einem Auslösen durch Überhitzen ein Austausch der Sicherung erfolgen muss. Außerdem ist kein redundanter Sicherheitsmechanismus vorhanden und bei Versagen der Schmelzsicherung droht eine Überhitzung der Behandlungsfläche über einen längeren Zeitraum. Des Weiteren werden Temperaturen ab 60 °C oder darüber hinaus, besonders über einen längeren Zeitraum von einigen Sekunden oder länger, als sehr unangenehm empfunden und können zu Hautschädigungen führen. Dies kann mindestens dazu führen, dass der Behandlungserfolg gefährdet wird, weil Behandlungen aufgrund eines unangenehmen Hautgefühls durch die hohen Temperaturen frühzeitig abgebrochen werden und somit der Therapieerfolg gefährdet wird. Dem Gerät liegt der therapeutische Gedanke zu Grunde, durch Wärmeanwendung Keime zu zerstören und Reizerreger der Haut abzutöten. Die herfür benötigten Behandlungsdauern und/oder-temperaturen sind allerdings nicht geeignet, Juckreiz durch gezielte Stimulation bestimmter Rezeptoren und die Modifikation des Immunsystems nachhaltig zu lindern. Temperaturen unterhalb von 42 °C sind wiederum ungeeignet, über eine Wärmeempfindung hinaus Effekte therapeutischer Art zu erzielen.

US 2011/0184502 A1 beschreibt ein Heizkissen für diverse, teils medizinische Anwendungen, welches Temperaturen von 38 °C - 71 °C über mindestens mehrere Minuten elektrisch erzeugt. Es werden nicht rückstellende Thermosicherungen in Serie als redundantes Sicherheitsfeature vorgeschlagen. Somit ist zwar ein redundanter Sicherheitsmechanismus vorhanden, dieser ist jedoch nicht reversibel und muss nach dem Auslösen ausgetauscht werden. Ein zweiter Nachteil bei der Verwendung von thermischen Sicherungen ist, dass diese erst bei Anliegen einer Temperatur oberhalb eines Schwellenwerts abschmelzen. Somit reagieren thermische Sicherungen erst bei Anliegen dieser kritischen Temperatur nach einer gewissen Reaktionszeit und somit gegebenenfalls zu spät im Vergleich zu einer Schmelzsicherung. Eine Schmelzsicherung löst bereits bei einem elektrischen Strom oberhalb eines Schwellenwertes aus, der eine zu hohe Temperatur verursachen könnte, wenn er zu lange fließt. Darüber hinaus ist sowohl der Temperaturbereich als auch die Dauer des Heizprozesses für eine Vielzahl von Anwendungen sicherlich relevant, jedoch ungeeignet, Pruritus durch Hitzeanwendung nachhaltig zu lindern.

Es wäre somit wünschenswert eine Vorrichtung bereitstellen zu können, welche die Vorzüge der hyperthermischen Behandlung für die Vielzahl der genannten Erkrankungen umsetzt und gleichzeitig Sicherheitsrisiken minimiert, bevorzugt indem ein redundanter, aber auch praktischer und hohen Standards genügender Sicherheitsmechanismus gegen Überhitzen verwendet wird.

### Aufgabe der Erfindung

Eine Aufgabe der Erfindung war es somit eine Vorrichtung zur hyperthermischen Behandlung bereitzustellen, welche die Nachteile des Standes der Technik beseitigt. Insbesondere war es eine Aufgabe der Erfindung eine Vorrichtung zur hyperthermischen Behandlung von Juckreizerkrankungen zu konstruieren, welche eine hohe Sicherheit mit einfachen Konstruktionselementen gewährleistet.

### Zusammenfassung der Erfindung

Die erfindungsgemäße Aufgabe wird durch eine Vorrichtung gemäß dem unabhängigen Patentanspruch 1 gelöst. Die abhängigen Patentansprüche stellen bevorzugte Ausführungsformen der Erfindung dar.

In einer bevorzugten Ausführungsform betrifft die Erfindung eine Vorrichtung zur hyperthermischen Behandlung von Juckreiz umfassend
a) mindestens eine Behandlungsfläche
   und
b) eine Regelvorrichtung zur Regulation der Temperatur der Behandlungsfläche,
   wobei die Regelvorrichtung die Behandlungsfläche durch Erhitzen mindestens eines Heizelementes in einer Aufheizphase auf eine Behandlungstemperatur zwischen 42 °C und 56 °C regulieren kann und die Behandlungstemperatur in einer Behandlungsphase für einen Zeitraum von 2 sec bis 12 sec gehalten werden kann,
   wobei zusätzlich ein hardwareimplementierter Temperaturwächter die Maximaltemperatur der Behandlungsfläche auf einen Wert innerhalb einer Toleranzspanne um 56 °C von weniger als ± 10% begrenzt und eine Schmelzsicherung bei Kurzschluss oder ungeregeltem Durchheizen die Stromversorgung der Vorrichtung abschaltet.

Unter der "hyperthermischen Behandlung von Juckreiz" wird im Sinne der Erfindung bevorzugt eine Behandlung von Erkrankungen verstanden, welche in der Regel mit dem Auftreten von Juckreiz einhergehen. Wie bereits ausgeführt, umfasst dies insbesondere die Behandlung von Juckreiz, welcher nach einem Stechen durch Insekten oder dem Kontakt mit giftigen Nesseltieren oder Pflanzen auftreten kann. Weiterhin wird unter der hyperthermischen Behandlung von Juckreiz ein bevorzugter Verwendungszweck der Vorrichtung verstanden, welcher bevorzugt die Behandlung von Herpeserkrankungen oder anderen Hautirritationen betrifft die zu einer Rötung, Schwellung oder anderen unangenehmen Symptomen führen, welche mit Juckreiz einhergehen. Juckreiz kann durch Parasiten genauso wie durch eine mechanische oder chemische (z.B. Umweltgifte oder Medikamente) Belastung bevorzugt der Haut hervorgerufen werden. Aber auch die Aufnahme einer bestimmten Nahrung oder eine Aufnahme von Endoparasiten, ebenso wie Autoimmunreaktionen, Hautpilz, Allergien, Xerodermie, Altersjucken, Winterjucken, Nieren- oder Lebererkrankungen, Stoffwechselstörungen, Tumore, Temperaturschwankungen, Wasserkontakt oder eine psychische Störung kann zu einem Juckreiz der Haut führen. Zur Linderung der Symptomatik dieser verschiedenen Erkrankungen, insbesondere des Juckreizes, wird die erfindungsgemäße Vorrichtung bevorzugt auf die betroffenen Hautpartien aufgelegt. Nach der Kontaktierung der Hautpartie mit der Behandlungsfläche sorgt eine Regelvorrichtung dafür, dass eine erfindungsgemäße Regulation der Temperatur der Behandlungsfläche erfolgt. Bevorzugt wird die Behandlungsfläche hierzu zunächst in einer Aufheizphase auf eine Behandlungstemperatur zwischen 42 °C und 56 °C geführt. Es ist bevorzugt, dass die Aufheizphase keinen längeren Zeitraum bedarf. Bevorzugt sollte die Aufheizphase nicht mehr als 10 s, besonders bevorzugt nicht mehr als 3 s betragen. Im Anschluss an die Aufheizphase wird die Temperatur der Behandlungsfläche bevorzugt bei der vorbestimmten Behandlungstemperatur gehalten. Die Behandlungstemperatur entspricht bevorzugt einer konstanten Temperatur, welche im genannten Bereich zwischen 42 °C und 56 °C liegt.

Diese Behandlungstemperatur wird während der Behandlungsphase bevorzugt konstant gehalten. Es kann aber auch bevorzugt sein, dass die Behandlungstemperatur nicht konstant gehalten wird. Beispielsweise kann die Behandlungsfläche auch in einer Temperaturrampe auf eine Maximaltemperatur im Bereich der Behandlungstemperatur zwischen 42 °C und 56 °C geführt werden. Im Anschluss daran kann es bevorzugt sein, dass die Temperatur kurzzeitig unterhalb des Bereiches der Behandlungstemperatur gesenkt wird. Anschließend kann die Temperatur in einer Rampe wieder steigen. Diese Vorzugsvariante hat sich bei einigen zu Juckreiz führenden Hauterkrankungen überraschenderweise als vorteilhaft gegenüber dem Beibehalten einer konstanten Behandlungstemperatur erwiesen. So kann es beispielsweise von Vorteil sein, die Maximaltemperatur nur für sehr kurze Zeit über eine ansteigende Temperatur zu erreichen und danach wieder auf einer Rampe etwas abzukühlen.

Die Behandlungsphase bezeichnet bevorzugt den Zeitraum, bei welchem die Temperatur im Bereich der Behandlungstemperatur von 42 °C bis 56 °C ist. Besonders bevorzugt dauert die Behandlungsphase zwischen 2 s und 12 s, ganz besonders bevorzugt zwischen 3 s und 6 s. Es ist besonders bevorzugt, dass die Behandlungsphase einen zusammenhängenden Zeitraum bezeichnet. Es kann aber auch sein, dass die Behandlungsphase durch das Führen der Temperatur in einer Rampe kurzzeitig unterbrochen wird. In diesem Falle wird als Zeitraum der Behandlungsphase bevorzugt diejenige Zeit verstanden, während welcher die Temperatur der Behandlungsfläche im Bereich der Behandlungstemperatur von 42 °C bis 56 °C liegt.

Durch die Regulation der Behandlungsfläche auf einer Temperatur zwischen 42 °C und 56 °C für eine Behandlungsphase zwischen 2 s und 12 s, bevorzugt zwischen 4s und 6 s, wird ein Wärmimpuls generiert, welcher es erlaubt eine wohldefinierte Wärmemenge auf die Hautstelle auf kontrollierte Weise zu bringen. Hierdurch kann vorteilhafterweise z.B. der Juckreiz, welcher durch einen Insektenstich beispielsweise durch eine Wespe oder Biene auftritt, überraschend wirksam gelindert werden. Zum einen resultiert die Linderung aus einer thermischen Neutralisation der Gifte der Insekten. Zum anderen bewirkt der Wärmeimpuls eine Nervenstimulation, welche die subjektive Wahrnehmung eines Juckreizes an den betreffenden Stellen stark reduziert. Die Wärmeübertragung führt überraschend zu einer Überlagerung des Juckreizes durch andere temperaturabhängige Hautempfindungen. Entgegen konventionellen Methoden zur Behandlung von Pruritus, welche das Juckempfinden über eine Regulation der Schmerzrezeptoren anvisieren, werden durch die bevorzugte Wärmebehandlung die freien Nervenenden der C-Fasern aktiviert. Die C-Fasern bezeichnen insbesondere die langsam leitenden Nervenfasern des somatosensiblen Systems und sind für die Schmerzwahrnehmung verantwortlich. Hierbei kommt insbesondere den freien Enden der C-Fasern, welche auch als Nozizrezeptoren bezeichnet werden, eine wichtige Rolle zu. Die Nervenenden der Fasern werden durch Gewebshormone (z. B. Histamin, Serotonin, Substanz P) aktiviert. Auch könnten Mastzellen in der Nähe der Nervenenden durch Ausschüttung des Mediators Tryptase an dem Prozess beteiligt sein. Insbesondere wird die Erkenntnis über den Wirkmechanismus bei Pruritus ausgenutzt, um durch eine Wärmebehandlung in überraschender Weise die Sinneswahrnehmung, welche durch die Fasern ausgelöst werden, zu regulieren. Neben dem besonders bevorzugten Einsatz der Vorrichtung zur Behandlung von Juckreiz bei Insektenstichen erlauben die genannten Zeiträume und Temperaturparameter eine Behandlung von Herpeserkrankungen und auf überraschender Weise auch weiteren Juckreizerkrankungen z.B. nach Kontakt mit giftigen Nesseltieren oder Pflanzen, wie Brennnesseln.

Eine weitere bevorzugte Variante verwendet eine Behandlungstemperatur zwischen 42 °C und 56 °C und besonders bevorzugt zwischen 50 °C und 53 °C. Es hat sich völlig überraschend gezeigt, dass mit den vorgenannten Parametern ein Juckreiz besonders stark reduziert werden kann. Eine Behandlungstemperatur zwischen 42 °C und 56 °C und insbesondere die bevorzugte Behandlungstemperatur zwischen 50 °C und 53 °C erlaubt eine Wirkung auf die Hautpartien, welche den Juckreiz schnell und effektiv lindern.

Insbesondere wurde erkannt, dass eine besonders starke Überlagerung des Juckempfindens erreicht werden kann, wenn in den betreffenden Hautpartien lokal die Thermo- und Capsaicinrezeptoren TRPV1 und TRPV2 zeitgleich aktiviert werden.

Der TRPV1 ist bei akutem Hitze-induzierten Schmerz in gesunder Haut beteiligt und reguliert beispielsweise das Heißempfinden bei Temperaturen um 45 °C bis 50 °C. Bei besonders starken schmerzhaften Hitzereizen, welche ab Temperaturen von über 52 °C eintreten wird darüber hinaus der TRPV2 aktiviert. Die Aktivierungsschwelle des TRPV1 liegt zwischen 40 °C und 45 °C, wohingegen die des TRPV2 zwischen 50 °C und 53 °C beträgt (Yao et al 2011, Somogyi et al. 2015, Cohen et al. 2014, Mergler et al. 2014).

Während durch aktuelle Forschungsergebnisse in der Literatur ein erstes Verständnis über die Wirkungsweise der TRPV1 und TRPV2 Rezeptoren als Temperatursensoren aufkommt, ist deren Rolle in der Empfindung von Juckreizen unbekannt. Ein Fachmann würde daher auch unter Kenntnis der Literatur nicht davon ausgehen, dass gerade die Aktivierung dieser Rezeptoren eine besonders effektive Überlagerung des Juckempfindens ermöglicht. Dies ist eine überraschende Erkenntnis, welche insbesondere in der besonders bevorzugt genannte Ausführungsform, welche eine Temperaturregelung der Behandlungsfläche in einem engen Bereich zwischen 50 °C und 53 °C vorsieht, genutzt wird. Eine Temperaturregelung der Behandlungsfläche in einem engen Bereich zwischen 50 °C und 53 °C erlaubt auf überraschend effektive Weise eine zeitgleiche Aktivierung der Rezeptoren, ohne unangenehm starke Schmerzempfindungen bei den behandelten Personen auszulösen. Durch experimentelle Versuche konnte der Bereich der Aktivierungsschwelle des TRPV2 als besonders optimierter Wirkbereich ermittelt werden. Hierbei kommt es voraussichtlich zu einem Feedbackmechanismus zwischen den Rezeptoren, welcher den Juckreiz besonders effektiv überlagert, ohne Nebenwirkungen zu verursachen. Die bevorzugte Behandlung der Hautpartien führt zu einem Abmildern des Juckempfindens, welches überraschenderweise noch Stunden nach der Behandlung anhält. Die langanhaltende Wirkungsweise der bevorzugten Ausführungsform ist zu mindestens teilweise auf eine Immunregulation durch die Wärmübertragung zurückzuführen. So wird nicht nur das Schmerzempfinden überlagert, sondern durch eine Regulation des Immunsystems wird die lokale Reizung der Haut aktiv unterdrückt. Vorteilhafterweise kann daher eine einmalige Behandlung bereits zu einem nachhaltigen Nachlassen des Juckempfindens führen. Es kann aber auch bevorzugt sein, mehrmals in zeitlicher Abfolge eine Behandlung durchzuführen. Die intervallartige Übertragung der Wärme mit einer Behandlungsphase von 2 s - 12 s oder besonders bevorzugt 4 s bis 6 s erreicht eine optimale Wirkung auf die Signalwege des Juckreizes ohne unerwünschte Nebenwirkungen auszulösen.

Im Sinne der Erfindung bezeichnet die Behandlungsfläche bevorzugt diejenige Fläche der Vorrichtung, welche während der Behandlung auf die Behandlungstemperatur aufgeheizt wird und in direktem thermischen Kontakt mit der Hautpartie steht. Die Behandlungsfläche kann eine zusammenhängende Fläche darstellen. Es kann auch bevorzugt sein, dass die Behandlungsfläche aus mehreren nicht zusammenhängenden Teilflächen besteht. Die Größe der Behandlungsfläche hängt bevorzugt von der Erkrankung und der Größe der von den Symptomen der Juckreizerkrankung betroffenen Hautpartien ab. Bei Insektenstichen ist die Größe der Behandlungsfläche zwischen 10 mm² und 100 mm² besonders bevorzugt zwischen 20 mm² und 60 mm² bevorzugt. Bei der Behandlung von Herpes beträgt die Behandlungsfläche bevorzugt zwischen 10 mm² und 80 mm² besonders bevorzugt zwischen 20 mm² und 50 mm². Weiterhin ist es besonders bevorzugt, dass die Behandlungsfläche für diese kleineren Hautpartien kreisförmig ist. Durch die so gewählten Größen und Geometrien der Behandlungsfläche kann eine der Ursache optimal angepasste Behandlung erfolgen, welche Effizienz und Wohlbefinden optimiert und so zu einem nachhaltigeren Behandlungserfolg beiträgt. Für die Behandlung von großflächigen Hauterkrankungen, welche mit Juckreiz einhergehen, beispielsweise nach Kontakt mit giftigen Quallen, können auch Behandlungsflächen von 1 cm² bis 18 cm², bevorzugt zwischen 6 cm² und 9 cm² bevorzugt sein. Bislang ging die Fachwelt davon aus, dass bei einer großflächigen Behandlung betroffener Hautpartien, eine positive Linderung des Juckreizes durch starke negative Nebenwirkungen wie Hautverbrennung oder hyperthermisches Schmerzempfinden überlagert werden. Es wurde jedoch erkannt, dass mit einer größeren Behandlungsfläche von ca. 1 cm² bis 18 cm², bevorzugt zwischen 6 cm² bis 9 cm² auf überraschender Weise auch großflächige Hautpartien, welche von Pruritus betroffen sind, behandelt werden können. So ist es beispielsweise möglich im Falle von Hautauschlägen durch bequemes und einfaches Auflegen der Behandlungsfläche auf die entsprechenden Hautpartien, das Juckempfinden durch die Hitzeübertragung in eine erträgliche Schmerzempfindung zu überführen. Sekundäre Schädigungen der Haut, beispielsweise Wundbildungen durch starkes Kratzen, können so wirksam vermieden werden. Mit Vorrichtungen mit kleineren Behandlungsflächen wäre für eine Behandlung von großflächigen Hautpartien ein mehrmaliges Auflegen an unterschiedlichen Positionen notwendig. Aufgrund des Zeitversatzes kann hierdurch jedoch nicht derselbe Effekt erzielt werden.

Es kann auch bevorzugt sein, dass eine Behandlungsfläche zwischen 7 cm² und 18 cm² Verwendung findet. Äußere Reize chemischer, mechanischer oder physikalischer Natur, die Juckempfinden auslösen können, werden von drei unterschiedlichen Rezeptorzellen (Sinneszellen) wahrgenommen. Bei diesen Sinneszellen handelt es sich um so genannte offene Nervenendigungen, deren reizaufnehmende Strukturen in der Epidermis und der darunterliegenden Dermis lokalisiert sind, und deren Axone die Signale über wahrgenommene Reize ins Rückenmark weiterleiten. Bei diesen Sinneszellen sind die unmyelenisierten C-Fasern von besonderer Bedeutung. Deren rezeptive Strukturen befinden sich teilweise bis 0,1 mm unter der Hautoberfläche. Bei den C-Fasern unterscheidet man polymodale mechanisch und hitzesensitive Fasern und mechanisch insensitive C-Fasern, die aber auch durch Hitze stimuliert werden können. C-Fasern nehmen nicht nur pruritogene Reize war, sondern dienen auch als Nozizeptoren (Schmerzrezeptoren). In der Literatur wurde gezeigt, dass Hitzereize als Gegenreiz Juckempfinden unterdrücken können. Die einzelnen C-Fasern nehmen Reize eines bestimmten Areals der Haut wahr, wobei ein definiertes Hautareal von einer Sinneszelle innerviert wird. Diese Region wird als rezeptives Feld bezeichnet. Die rezeptiven Felder von C-Fasern können teilweise überlappend sein. Untersuchungen am Menschen durch so genanntes Micromapping haben gezeigt, dass die mechanisch insensitiven C-Fasern rezeptive Felder von bis zu 5 cm² Größe haben; die von mechanisch sensitiven C-Fasern sind etwas kleiner und bis zu 2 cm² groß. Bei der bevorzugten Behandlungsgröße zwischen 7 cm² und 18 cm² werden somit die rezeptiven Felder der unterschiedlichen C-Fasertypen auf überraschende Weise abgedeckt und darüber hinaus wird der Effekt der horizontal abfließenden Wärme kompensiert.

Die Größe der Behandlungsfläche bezieht sich bevorzugt jeweils auf die gesamte Kontaktfläche, über welche eine Hautpartie einen Wärmeimpuls erfährt. Im Falle einer Behandlungsfläche, welche aus mehreren Teilflächen besteht, entspricht die Größe der Behandlungsfläche bevorzugt der Summe der einzelnen Teilflächen. Eine solche Aufteilung in Teilflächen kann bei bestimmten Erscheinungsformen von Juckreiz verursachenden Leiden vorteilhaft sein, ebenso wie bei der Behandlung bestimmter Körperstellen.

Es ist bevorzugt, dass die Behandlungsfläche mit Hilfe von mindestens einem Heizelement auf die gewünschte Behandlungstemperatur gebracht wird. In einer bevorzugten Ausführungsform entspricht die Behandlungsfläche der Oberfläche einer Heizplatte, welche mit Hilfe eines Heizelementes erhitzt wird, wobei zum Beispiel ein Halbleiter-Bauelement zum Einsatz kommen kann. Die Behandlungsfläche kann jedoch auch eine homogene Materialfläche bezeichnen, welche durch mehrere Heizelemente temperiert wird. Beispielsweise kann es bevorzugt sein zwei oder vier Heizelemente zu verwenden, um die Behandlungsfläche besonders homogen und schnell auf die Behandlungstemperatur zu führen. Es kann auch bevorzugt sein eine Heizplatte umfassend ein Heizelement zu beschichten. In dem Fall wird unter der Behandlungsfläche bevorzugt die Beschichtung der Heizplatte verstanden. So gibt die Behandlungstemperatur bevorzugt stets jene Temperatur an, welche an der Hautpartie des Patienten vorliegt. Durch die je nach Einsatzgebiet bevorzugte Ausführungsform bezüglich der Kombination von Heizelement und Behandlungsfläche kann eine auf Effizienz, Kompaktheit und Behandlungserfolg hin optimierte Vorrichtung bereitgestellt werden.

Es bevorzugt, dass die Regelvorrichtung das Erhitzen des Heizelementes derart regulieren kann, dass die Behandlungstemperatur an der Behandlungsfläche vorliegt. So kann eine optimale Kontrolle der Behandlungstemperatur gewährleistet werden und ein ungewünschtes Überhitzen der Behandlungsfläche unterbunden werden.

Im Sinne der Erfindung ist die Regelvorrichtung bevorzugt ein Prozessor, ein Prozessorchip, Mikroprozessor oder ein Mikrokontroller, welcher konfiguriert ist, um die Temperatur der Behandlungsfläche mit Hilfe des mindestens einen Heizelementes gemäß der vorgegebenen Werte für die Behandlungstemperatur zu regulieren. Eine solche Regelvorrichtung zeichnet sich durch Kompaktheit, Zuverlässigkeit, Kosteneffizienz, geringen Stromverbrauch und hohe Regeleffizienz aus.

Das mindestens eine Heizelement ist ein Bauelement, für welche verschiedene Ausführungsformen aus dem Stand der Technik hinreichend bekannt sind. So kann das Heizelement einen Leistungswiderstand umfassen, bei welchem in Abhängigkeit des Stromflusses eine wohldefinierte Temperatur generiert wird. Bevorzugt kann zur quantitativen Steuerung des Stromflusses durch das Heizelement ein Feldeffekttransistor (FET) verwandt werden. Es kann aber auch bevorzugt sein, einen FET selbst als Heizelement einzusetzen. Hierbei wird Energiedissipation im Transistor selbst genutzt, um Wärme zu generieren und die Behandlungsfläche auf die Behandlungstemperatur zu bringen. FETs sind als Heizelemente besonders bevorzugt, da diese aufgrund ihrer geringen Größe eine kleine Dimensionierung erlauben. Weiterhin sind FETs besonders reaktiv und gewährleisten durch eine sehr dynamische Wärmeerzeugung und Wärmeabgabe ein besonders schnelles Ansprechverhalten der Heizelemente.

Bevorzugt kann die Regelvorrichtung durch die Vorgabe der Stromzufuhr zum Heizelement kontrollieren, welche Temperatur an der Behandlungsfläche vorliegt. Beispielsweise kann mit Hilfe einer Kalibration die Korrelation zwischen Stromfluss und/oder Spannung an dem Heizelement mit der Temperatur an der Behandlungsfläche ermittelt werden, sodass auf Basis der Kalibrierung stets eine gewünschte Behandlungstemperatur zwischen 42 °C und 56 °C eingestellt werden kann. Es kann aber auch bevorzugt sein, die Behandlungstemperatur durch die Regelvorrichtung mit Hilfe einer Feedbackschleife zu regulieren. So kann es bevorzugt sein einen Temperatursensor einzusetzen, welcher die Temperatur der Behandlungsfläche misst, wobei die Regelvorrichtung anhand der Temperaturdaten die Stromzufuhr zum Heizelement reguliert. Zu diesem Zweck kann die Regelvorrichtung beispielsweise einen Mikroprozessor umfassen, welcher Messdaten auswerten und Stromparameter festlegen kann. So kann die Temperatur sehr effizient und zuverlässig gesteuert werden.

Besonders bevorzugt umfasst die Vorrichtung mindestens zwei weitere Sicherheitselemente, welche die Temperatur der Behandlungsfläche kontrollieren.

Zum einen umfasst die Vorrichtung einen hardwareimplementierten Temperaturwächter, welcher die Maximaltemperatur der Behandlungsfläche auf einen Wert zwischen 54 °C und 58 °C bevorzugt ungefähr 56 °C begrenzt. Die Maximaltemperatur bezeichnet bevorzugt jene Temperatur, welche die Behandlungsfläche maximal während der Behandlungsphase erreicht. Der hardwareimplementierte Temperaturwächter erlaubt vorteilhafterweise eine Sicherstellung, dass die Maximaltemperatur einen Wert zwischen 54 °C und 58 °C bevorzugt von ungefähr 56 °C nicht überschreitet. Im Sinne der Erfindung bezeichnen Angaben wie ungefähr, ca., nahezu oder synonyme Begriffe bevorzugt eine Toleranzspanne von weniger als ± 10%, bevorzugt weniger als ± 5% besonders bevorzugt weniger als ± 1%. Im Sinne der Erfindung bezeichnet ein "hardwareimplementierte Temperaturwächter" bevorzugt ein Temperaturkontrollsystem für die Behandlungsoberfläche, welches hardwarebasiert die Stromversorgung der Heizelemente für die Behandlungsfläche abschalten kann. Insbesondere erlaubt der "hardwareimplementierte Temperaturwächter" bevorzugt, dass eine Abschaltung der Stromversorgung der Heizelemente bei Überschreitung der Maximaltemperatur unabhängig von der Regulation der Heizelemente durch die Regelvorrichtung, also z.B. dem Mikroprozessor erfolgt. Sollte zur Regulation der Heizelemente beispielsweise auf der Regelvorrichtung eine Firmware installiert vorliegen, so ist es bevorzugt, dass der hardwareimplementierte Temperaturwächter auch bei einem Ausfall oder einem fehlerhaften Ausführen der Firmware die Maximaltemperatur der Behandlungsfläche zuverlässig begrenzt.

Hierdurch kann durch einfache konstruktive Mittel besonders effektiv sichergestellt werden, dass die Behandlungsfläche der Vorrichtung eine Maximaltemperatur nicht überschreitet. Selbst unter Auftreten von Fehlsteuerungen in der Regelvorrichtung, beispielsweise nach dem Eintreten von Flüssigkeiten, kann aufgrund des hardwarebasierten Temperaturwächters vorteilhafterweise jederzeit gewährleistet werden, dass die Behandlungsfläche eine Maximaltemperatur von einem Wert zwischen 54 °C und 58 °C bevorzugt von ungefähr 56 °C nicht überschreitet. Durch dieses zusätzliche technische Element zur Temperaturüberwachung ist es möglich einen ausgezeichneten Sicherheitsstandard zu wahren, ohne mit der Funktionsweise der Vorrichtung zur hyperthermischen Behandlung zu interferieren.

Es hat sich überraschenderweise gezeigt, dass durch eine Maximaltemperatur von einem Wert zwischen 54 °C und 58 °C bevorzugt von ungefähr 56 °C weder der Behandlungserfolg durch die hyperthermische Behandlung gefährdet wird, noch ein unangenehmes Empfinden auf der Haut erzeugt wird, so dass diese Maximaltemperatur eine ideale erste Sicherheitsstufe zum Schutz vor Überhitzen darstellt.

Als weiteres Sicherheitselement weist die erfindungsgemäße Vorrichtung eine Schmelzsicherung auf, welche bei einem Kurzschluss der Vorrichtung oder ungeregeltem Durchheizen der Vorrichtung die Stromversorgung zur Vorrichtung unterbricht. Im Sinne der Erfindung wird unter einer Schmelzsicherung bevorzugt eine Überstromschutzeinrichtung verstanden, bei welcher beispielsweise durch das Abschmelzen eines Schmelzleiters ein Stromkreis unterbrochen werden kann, sobald die Stromstärke einen Grenzwert über eine zu bestimmende Zeit überschreitet. Es ist bevorzugt, dass die Schmelzsicherung in der Vorrichtung zwischen der Einspeisung der Versorgungsspannung in die Vorrichtung und der Vorrichtung selbst vorliegt. Sollte es zu einem Störfall kommen, welcher dadurch gekennzeichnet ist, dass ein unkontrollierter hoher Strom von der Versorgungseinspeisung in die Vorrichtung fließt, schaltet die Schmelzsicherung vorteilhafterweise die komplette Spannungsversorgung der Vorrichtung ab. Eine Schmelzsicherung bietet zum einen ausreichend schnellen, zum anderen einen extrem zuverlässigen Schutz.

Es hat sich gezeigt, dass selbst unter fehlerfreier Konstruktion der Vorrichtung und dem Bereitstellen eines hardwareimplementierten Temperaturwächters es nicht ausgeschlossen werden kann, dass es aufgrund einer fehlerhaften Bedienung oder Schädigung der Vorrichtung in äußerst seltenen Fällen zu einem Durchheizen der Heizelemente kommt. Unter dem Durchheizen der Heizelemente wird im Sinne der Erfindung bevorzugt verstanden, dass die Temperatur der Heizelemente unkontrolliert, d.h. nicht durch eine temperaturbasierte Regelung mithilfe der Regelvorrichtung steigt. Falls in diesen Störfällen der hardwareimplementierte Temperaturwächter versagt, kann die Behandlungsfläche unkontrolliert auf Temperaturen weit über der gewünschten Behandlungstemperatur ansteigen, beispielsweise auf Temperaturen von weit über 65 °C.

Obwohl ein solches fehlerhaftes Durchheizen äußerst selten auftritt, kann dies zu schwerwiegenden Schädigungen beim Patienten führen. Dies liegt insbesondere daran, dass die hyperthermisch zu behandelnden Hautpartien zumeist besonders empfindlich sind und beispielsweise durch eine Rötung, Schwellung oder gar Wundbildung gekennzeichnet sind. Eine deutlich erhöhte Temperatur über 65° kann lokal an diesen Stellen in besonderen Maße zu starken Schmerzen und Hautverbrennungen führen.

Im Hinblick auf die besonderen Umstände der Nutzung der Vorrichtung und damit verbundenen Sicherheitsanforderungen ist die genannte Schmelzsicherung besonders vorteilhaft, um noch für den unwahrscheinlichsten Fall einer Störung eine Abschaltung des Heizens der Behandlungsfläche gewährleisten zu können. So wird mithilfe der Schmelzsicherung unabhängig von jeglicher Temperaturmessung, aufgrund z.B. fehlerhafter Temperatursensoren, ein übermäßiges Heizen der Behandlungsfläche unterbunden. Es wurde erkannt, dass die Stromversorgung der Vorrichtung eine zentrale Regulationsschnittstelle darstellt, welche höchsten Sicherheitsanforderungen genügt. Durch eine Integration der Schmelzsicherung in den Stromfluss zur Versorgung der Vorrichtung kann sichergestellt werden, dass ein maximaler Versorgungsstrom während einer bestimmten Zeit nicht überschritten wird. Da ein Durchheizen und ein unkontrolliertes Heizen der Heizelemente über die gewünschte Temperatur mit einem erhöhten Stromfluss zusammenhängt, kann hierdurch auf besonders zuverlässige Weise ein Überhitzen der Behandlungsfläche vermieden werden. Insbesondere kann durch die Stromkontrolle sehr schnell reagiert werden, bevor der Strom so lange wirkt, dass er eine seiner Stärke entsprechende Temperatur erzeugt. Ein rein an der Temperatur orientierter letztinstanzlicher Sicherheitsmechanismus könnte außerdem aufgrund von thermischen Trägheiten der beteiligten Bauteile nicht schnell genug sein.

Bei der erfindungsgemäßen Vorrichtung zeigt sich eine besonders vorteilhafte und synergistische Wirkung der kombinierten Verwendung eines hardwareimplementierten Temperaturwächters und einer Schmelzsicherung.

So ist es ein Nachteil der Schmelzsicherung, dass diese nach dem einmaligen Auslösen eine permanente Abkopplung der Versorgungsspannung von der Vorrichtung zur Folge hat. Eine erneute Inbetriebnahme der Vorrichtung nach dem Auslösen der Schmelzsicherung bedarf der Reparatur durch einen Techniker, beispielsweise den Austausch der Schmelzsicherung. Im Hinblick auf die Kosten ist die Vorrichtung in der Regel bei einem Auslösen der Schmelzsicherung unbrauchbar geworden.

Vorteilhafterweise ist der hardwareimplementierte Temperaturwächter jedoch so eingestellt, dass es nicht zu einem permanenten Abschalten der Stromversorgung der Vorrichtung kommen muss. Vielmehr ist der hardwareimplementierte Temperaturwächter derart konzipiert, dass wenn die Temperatur der Behandlungsfläche eine Maximaltemperatur überschreitet, während des Zeitraumes des Überschreitens die Stromversorgung der Heizelemente unterbrochen wird. Die Stromunterbrechung durch den hardwareimplementierten Temperaturwächter ist somit vorteilhafterweise reversibel, d.h. sobald die Temperatur der Behandlungsfläche wieder unter die Maximaltemperatur fällt, können die Heizelemente wieder heizen.

So kann auch nach einem einmaligen Auftreten einer Fehlsteuerung die bestimmungsgemäße Benutzung der Vorrichtung fortgeführt werden. Der Benutzer würde gegebenenfalls nichts von der Fehlsteuerung bemerken, da durch die gewählte Maximaltemperatur, die Effektivität und die Unabhängigkeit des hardwareimplementierten Temperaturwächters keine für den Benutzer als unangenehm empfundenen Temperaturen entstehen und die Vorrichtung bei der nächsten Benutzung im Falle einer einmaligen Fehlsteuerung wieder einwandfrei funktioniert könnte.

Die Kombination der Sicherheitsmerkmale eines hardwareimplementierten Temperaturwächters mit einer Schmelzsicherung erlauben eine überraschend zuverlässige Kontrolle der Temperatur mit möglichst wirtschaftlichen Mitteln aufgrund gestaffelter Sicherheitsschranken.

Ein weiterer synergistischer Effekt durch die Kombination der Sicherheitsmerkmale eines hardwareimplementierten Temperaturwächters mit einer Schmelzsicherung kann darin gesehen werden, dass beispielsweise ein zwar unwahrscheinliches, jedoch mögliches einmaliges Versagen der Regelvorrichtung durch den hardwareimplementierten Temperaturwächter reversibel abgefangen wird. Sollte jedoch ein äußerst unwahrscheinliches größeres Problem auftreten, welches den hardwareimplementierten Temperaturwächter umfasst, so tritt die Schmelzsicherung als letztinstanzliche Absicherung in Aktion. Da diese jedoch irreversibel ist, kann keine unter diesen Umständen gefährliche Weiterbenutzung durch den Benutzer stattfinden, sondern es wird ein Gang zum Techniker bzw. zum Fachhandel veranlasst.

Bevorzugt erfolgt bereits mithilfe der Regelvorrichtung eine Temperierung der Behandlungsfläche. Falls die Regelvorrichtung aufgrund z.B. einer fehlerhaften Elektronik versagt, erlaubt der hardwareimplementierte Temperaturwächter ein Abschalten der Heizelemente unabhängig von der Regelvorrichtung. Selbst bei einer solchen Störung der Regelvorrichtung würde eine Schmelzsicherung nicht ausgelöst. Nur in dem äußerst seltenen Fall, dass sowohl die Regelvorrichtung, als auch der hardwareimplementierte Temperaturwächter versagt, beispielsweise bei einer Beschädigung der entsprechenden Bauelemente, gewährleistet die Schmelzsicherung ein finales Kontrollelement. Kommt es zu einem erhöhten Strombedarf der Heizelemente aufgrund eines starken Erhitzens, schaltet die Schmelzsicherung die gesamte Stromversorgung der Vorrichtung ab. Durch diese Staffelung der Sicherheitsmechanismen kann eine einmalige Störung der Regelvorrichtung äußerst sicher abgefangen werden. Der hardwareimplementierte Temperaturwächter schreitet unbemerkt und schnell ein, ohne die Benutzbarkeit der Vorrichtung zu beeinflussen. Durch die nachgeschaltete Schmelzsicherung kann ein noch höheres Sicherheitslevel erreicht werden, so dass dem Benutzer eine ungemein effektive und sichere Behandlungsvorrichtung zur Verfügung gestellt werden kann.

Durch eine Hintereinanderschaltung der Sicherheitsschranken kann überraschend gewährleistet werden, dass die Behandlungsfläche nicht in einen Temperaturbereich gelangt, welcher den Patienten gefährden könnte.

In einer bevorzugten Ausführungsform der Erfindung umfasst die Vorrichtung mindestens einen ersten Temperatursensor zur Messung der Temperatur der Behandlungsfläche, wobei die Regelvorrichtung basierend auf den Messdaten des Temperatursensors die Temperatur des mindestens einen Heizelementes einstellt. Durch einen solchen Temperatursensor kann die die Temperatur der Behandlungsfläche sehr zuverlässig durch die Regelvorrichtung gesteuert werden.

Im Sinne der Erfindung ist ein Temperatursensor bevorzugt ein elektrisches oder elektronisches Bauelement, welches in Abhängigkeit der Temperatur am Sensor ein elektrisches Signal generiert. Im Stand der Technik sind eine Vielzahl von Temperatursensoren bekannt wie beispielsweise Halbleiter-Temperatursensoren, Widerstandstemperatursensoren, pyroelektrische Materialien, Thermoelemente oder Schwingquarze. Die Regelvorrichtung ist weiterhin bevorzugt derart konfiguriert, dass diese die Messwerte der Temperatursensoren aufnehmen und auswerten kann, um eine Regulation der Heizplatten zu bewirken. Die Regulation der Heizplatten kann bevorzugt mithilfe des Anliegens eines elektrischen Stroms oder einer Spannung erfolgen. Es ist besonders bevorzugt, dass der Temperatursensor die Temperatur der Behandlungsfläche direkt misst, d.h. dass der Temperatursensor in Kontakt mit der Behandlungsfläche ist, wobei sich der Temperatursensor sowohl auf der inneren Seite der Behandlungsfläche, als auch auf der äußeren Seite der Behandlungsfläche befinden kann oder aber in die Behandlungsfläche implementiert ist.

Es kann aber auch bevorzugt sein, dass der Temperatursensor nicht die Behandlungsfläche direkt, sondern die Heizelemente oder einen Materialpunkt zwischen den Heizelementen und der Behandlungsfläche kontaktiert und überwacht. Im Falle von mehreren Heizelementen, welche die Behandlungsfläche erhitzen, kann es beispielsweise auch bevorzugt sein den Temperatursensor zwischen den Heizelementen zu platzieren. Aus den Messdaten für die Temperatur über die Heizelemente oder einem Messpunkt in einer bestimmten Distanz zur Behandlungsfläche kann ebenfalls auf die Temperatur der Behandlungsfläche geschlossen werden. Im Sinne der Erfindung ist es bevorzugt, dass die Temperatur der Behandlungsfläche die durchschnittliche Temperatur der Behandlungsfläche meint.

Eine Auswertung der Temperatur der Behandlungsfläche erlaubt eine besonders präzise Regulation des mindestens einen Heizelementes, um eine optimale Temperaturverteilung auf der Behandlungsfläche und somit Wärmeübertragung an die zu behandelnden Hautpartien sicherzustellen. Insbesondere im Hinblick auf die vielfältigen Anwendungsmöglichkeiten der Vorrichtung zur Behandlung verschiedener Erkrankungen, welche mit Juckreiz einhergehen können, ist eine temperaturbasierte Feedback-Regulation mit Hilfe der Regelvorrichtung geeignet, um eine zuverlässige hyperthermische Behandlung mit optimalen Temperaturwerten durchzuführen.

In einer bevorzugten Ausführung der Erfindung umfasst der hardwareimplementierte Temperaturwächter mindestens einen zweiten Temperatursensor zur Messung der Temperatur der Behandlungsfläche und einen Komparator, wobei der Komparator die Temperatur der Behandlungsfläche mit der Maximaltemperatur vergleicht und bei Überschreiten der Maximaltemperatur die Stromzufuhr zu dem mindestens einen Heizelement unterbindet. Im Sinne der Erfindung bezeichnet ein Komparator bevorzugt eine elektronische Schaltung zum Vergleichen zweier Spannungen, wobei am Ausgang in binärer Weise angezeigt wird, welcher der beiden Spannungen höher ist. Im Stand der Technik sind hinreichend verschiedene Komparatoren bekannt, welche geeignet sind aus zwei analogen Spannungen ein binäres Ausgangsignal auszugeben, welche aussagt, welche der Eingangsspannung höher ist. Als Beispiel für eine Komparator-Schaltung sei der Schmitt-Trigger genannt. Es ist bevorzugt, dass an einem Eingang des Komparators mit Hilfe eines Spannungsverteilers ein Referenzwert für eine Spannung angelegt wird. Dieser Referenzwert entspricht bevorzugt dem Spannungswert, welcher der zweite Temperatursensor aufweisen würde, wenn die Temperatur der Behandlungsfläche gleich der Maximaltemperatur ist. An dem zweiten Eingang des Komparators liegt bevorzugt die Ausgangsspannung des Temperatursensors an, welche von der Temperatur der Behandlungsfläche abhängt. Ein besonders bevorzugter Temperatursensor umfasst zu diesem Zweck einen NTC-Thermistor, d.h. einen Heißleiter. Dieser besitzt einen negativen Temperaturkoeffizienten, sodass bei einer Erhöhung der Temperatur der Widerstand fällt und ein höherer Strom fließt. Es können aber auch Kaltleiter, d.h. PTC-Thermistoren verwandt werden, welche einen positiven Temperaturkoeffizienten besitzen, sodass bei einer Erhöhung der Temperatur der Widerstand steigt und ein niedrigerer Strom fließt.

Steigt die Temperatur der Behandlungsfläche, bewegt sich der durch den zweiten Temperatursensor gesteuerte Spannungswert am Komparator auf den Referenzwert der Spannung zu, welcher der Maximaltemperatur entspricht. Sobald die Temperatur die Maximaltemperatur überschreitet, ändert sich das Ausgangsignal am Komparator binär. Der Komparator ist bevorzugt in die Stromversorgung der Heizelemente integriert. D.h. bevor die Temperatur der Behandlungsfläche die Maximaltemperatur erreicht, gibt der Komparator bevorzugt die Versorgungsspannung der Heizelemente frei. Sobald jedoch die Temperatur höher als die Maximaltemperatur ist, schaltet der Ausgang des Komparators ab und unterbricht die Versorgungsspannung der Heizelemente. Fällt die Temperatur der Behandlungsfläche wieder, wird die Versorgungsspannung vorteilhafterweise wieder durch den Komparator freigegeben. Hierdurch kann ein reversibles An- und Abschalten der Heizelemente nur für den Zeitraum erfolgen, während die Temperatur der Behandlungsfläche die Maximaltemperatur überschreitet. Weiterhin kann es bevorzugt sein, dass der Komparator durch die Regelvorrichtung beim Starten der Vorrichtung freigeschaltet wird. Erfolgt somit kein ordnungsgemäßer Start der Vorrichtung ist der Komparator in der Voreinstellung so konfiguriert, dass die Spannungsversorgung der Heizelemente unterbrochen ist.

Die beschriebene bevorzugte Ausführungsform des hardwareimplementierten Temperaturwächters hat sich in Tests als besonders robust und zuverlässig erwiesen. Aufgrund der Reversibilität der Sicherheitsabschaltung und einfachen Konstruktionsweise zeichnet sich die bevorzugte Ausführungsform weiterhin durch niedrige Herstellungs- und Wartungskosten aus.

Durch die von der Regelvorrichtung unabhängigen Bauweise samt eigenem Temperatursensor kann ein zuverlässiger Betrieb auch bei Ausfall eines Bauteils der Regelvorrichtung gewährleistet werden.

Ebenso ist ein hardwareimplementierter Temperaturwächter in beschriebener Form unter Ausnutzung eines Komparators besonders schnell, da Komparatoren weit verbreitete elektronische Bauteile sind, die sich neben ihrer Zuverlässigkeit durch ihre schnelle Schaltfähigkeit ausweisen. So gibt es beispielsweise Komparatoren mit Schaltzeiten von ns und darunter. Es konnte überraschenderweise festgestellt werden, dass durch den Einsatz von Komparatoren in der Schaltung aufgrund Ihrer Schnelligkeit ein besonders wirksamer Schutzmechanismus gegen Überhitzen der Behandlungsfläche aufgebaut werden konnte.

In einer bevorzugten Ausführungsform der Erfindung ist die Vorrichtung dadurch gekennzeichnet, dass die Schmelzsicherung einen Schwellenwert für einen maximalen Strom aufweist, welcher der Erhitzung der Behandlungsfläche auf einen Wert zwischen 65 °C und 70 °C bevorzugt von 65 °C für 1 Sekunde entspricht. Tests haben gezeigt, dass erst eine Temperaturerhöhung von über 65 °C für länger als 1 Sekunde sehr kritisch für das Schmerzempfinden ist und zu Schädigungen der Hautpartien führen kann. Vorteilhafterweise wird durch Einstellung der Schmelzsicherung auf diese Parameterwerte die Schmelzsicherung nicht vorzeitig bei unkritischen Temperaturerhöhungen der Behandlungsfläche ausgelöst. Hierdurch kann Wirtschaftlichkeit erhöht werden, ohne einen Kompromiss in Bezug auf die Sicherheit einzugehen. Der Fachmann weiß anhand der elektrischen Parameter der Heizelemente, welche Schmelzsicherung gewählt werden sollte, um die angebenden Werte zu gewährleisten. Hierzu kann auch eine Messung der Stromzufuhr bei gleichzeitiger Messung der Temperatur der Behandlungsfläche erfolgen. Weiterhin ist es besonders bevorzugt eine flinke Schmelzsicherung zu verwenden, welche auf eine Stromerhöhung innerhalb von bevorzugt weniger als 20 ms reagiert. So wurde erkannt, dass auch eine kurzfristige Stromerhöhung von weniger als 20 ms aufgrund der thermischen Trägheit der Behandlungsfläche zu einer Temperaturerhöhung von länger als 1 Sekunde führen kann.

Gegenüber nicht rückstellenden, rein temperaturabhängigen Thermosicherungen, welche ebenfalls durch Abschmelzen funktionieren, hat die hier verwendete stromabhängige Schmelzsicherung einige Vorteile. Bei nicht rückstellenden, reinen temperaturabhängigen Thermosicherungen geschieht das Abschmelzen nicht bei Anliegen eines Stromes oberhalb eines Schwellenwertes, sondern erst bei Anliegen einer externen Temperatur, welche größer ist als eine definierte Maximaltemperatur. Gegenüber nicht rückstellenden reinen temperaturabhängigen Thermosicherungen können stromabhängige Schmelzsicherungen somit schon reagieren, bevor in Folge eines länger wirkenden erhöhten Stroms eine bestimmte unerwünschte Temperatur überhaupt erreicht wird. Ebenso brauchen nicht rückstellende reine temperaturabhängige Thermosicherungen bei Anliegen einer externen Temperatur, welche größer ist als eine definierte Maximaltemperatur, immer eine gewisse Reaktionszeit. Somit kann es zu gefährlichen, weiteren Temperaturerhöhungen kommen. Stromabhängige Schmelzsicherungen reagieren im Gegensatz dazu schneller und mit minimalen systembedingten Latenzzeiten.

In einer bevorzugten Ausführungsform der Erfindung ist die Vorrichtung dadurch gekennzeichnet, dass der Schwellenwert der Schmelzsicherung bevorzugt zwischen 1 A und 2,5 A besonders bevorzugt ungefähr 2 A beträgt. Tests haben gezeigt, dass in Bezug auf die bevorzugten Heizelemente die genannten Schwellenwerte besonders zuverlässig gewährleisten, dass die Temperatur der Behandlungsfläche eine Temperatur von 65 °C bis 70 °C für nicht länger als 1 Sekunde überschreitet. Durch das Schmelzen der Schmelzsicherung ab Stromwerten von 1 A bis 2.5 A kann somit sichergestellt werden, dass die Temperatur der Behandlungsfläche nicht in einen gesundheitsgefährdenden Bereich kommen kann. So kommt es bei einer regulären Behandlung zu einem regulären Behandlungsstrom, welcher unterhalb von 2,5 A, bevorzugt 1 A liegt. Tritt ein Fehler auf, z.B. bei einem Durchheizen, so fließt ein erhöhter Strom. In diese Falle Sicherung greift die Sicherung ein und verhindert wirksam ein unkontrolliertes Aufheizen.

Durch die vorteilhafte Auswahl der Maximaltemperatur des hardwareimplementierten Temperaturwächters auf einen Wert zwischen 54 °C und 58 °C bevorzugt von ungefähr 56 °C kann außerdem sichergestellt werden, dass der Abstand groß genug ist zur Temperatur beim Auslösen der Schmelzsicherung in Folge eines Stromwertes oberhalb des Schwellenwertes. So kann ein versehentliches Auslösen der Schmelzsicherung, welche mindestens einen Austausch der Sicherung zur Folge hätte, vermieden werden, so lange keine schwerwiegende Störung, welche den hardwareimplementierten Temperaturwächter einschließt, vorliegt.

In einer weiteren bevorzugten Ausführungsform der Erfindung ist die Vorrichtung dadurch gekennzeichnet, dass die Behandlungsfläche eine Dicke zwischen 0,2 mm und 5 mm, bevorzugt zwischen 0,5 mm und 2 mm, besonders bevorzugt zwischen 1 mm und 1,5 mm aufweist und aus einem Material besteht, welches eine Wärmeleitfähigkeit bei 50°C zwischen 20 W/mK und 400 W/mK, bevorzugt zwischen 100 und 350 W/mK aufweist. Die Wärmeleitfähigkeit (auch als Wärmeleitzahl bezeichnet) charakterisiert bevorzugt die thermischen Eigenschaften des Materials, aus welchem die Behandlungsfläche gefertigt ist. Die Wärmeleitfähigkeit gibt an, wie hoch die Wärmemenge ist, welche durch die Behandlungsfläche geleitet wird, wenn an dieser ein Temperaturgradient anliegt. Neben der Wärmeleitfähigkeit hängt der Wärmetransport von der Dicke der Behandlungsfläche, der Größe der Behandlungsfläche und dem Temperaturunterschied zwischen der Innenseite der Behandlungsfläche (Kontakt mit den Heizelementen) und der äußeren Seite der Behandlungsfläche (Kontakt mit der Haut) ab. Die Wärmeleitfähigkeit wird bevorzugt als Verhältnis der transportierten Wärmeleistung Watt (W) pro Temperaturdifferenz in Kelvin (K) und pro Meter (m) angegeben. Die Wärmeleitfähigkeit kann aber auch bevorzugt als Verhältnis der transportierten Wärmeleistung Watt (W) pro Temperaturdifferenz in Millikelvin (mK) angegeben werden. Da die Wärmeleitfähigkeit sich weiterhin in Abhängigkeit der Temperatur leicht ändern kann, wird vorliegend die Referenztemperatur mit 50 °C angegeben. Die Dicke der Behandlungsfläche bezeichnet weiterhin bevorzugt die Ausdehnung der Behandlungsfläche zwischen der äußersten Fläche, welche die Haut kontaktiert und der innersten Fläche, an welcher die Heizelemente anliegen.

Bei einer Dicke der Behandlungsfläche zwischen 0,2 mm und 5 mm, bevorzugt zwischen 0,5 mm und 2 mm und besonders bevorzugt zwischen 1 mm und 1,5 mm ergibt sich in Kombination mit der bevorzugten Wärmeleitfähigkeit zwischen 100 und 350 W/mK eine therapeutisch besonders wirkungsvolle Wärmeabgabe an die Haut. In experimentellen Versuchen haben sich die bevorzugt genannten Parameter als überraschend vorteilhaft erwiesen. So vermeidet eine derart konzipierte Behandlungsfläche eine zu schnelle Abgabe der Wärme an die betreffenden Hautpartien, wodurch ein unangenehm stechender Schmerz ausgelöst werden kann. Dennoch erfolgt die Wärmeabgabe in einem Zeitraum, welcher ausreichend impulshaft erfolgt, um effektiv die Rezeptoren anzuregen und einen Juckreiz zu überlagern. Die genannten Parameter stellen daher eine optimierte Auswahl dar, welche für einen Fachmann nicht nahelag. Außerdem wird durch die Parameter bevorzugt gewährleistest, dass die Wärme der Behandlungsfläche während der Behandlungsphase schnell und effektiv an die Hautpartie abgegeben wird, sodass ein Auftreten von Restwärme keine Gefährdung darstellt.

In einer bevorzugten Ausführungsform umfasst die Behandlungsfläche Keramik oder Gold. Es ist besonders bevorzugt, dass die Behandlungsfläche aus Gold oder Keramik besteht. Die Materialien Keramik und Gold fallen zum einen in den experimentell ermittelten, bevorzugten Bereiche der Wärmeleitfähigkeit. Weiterhin speichern die Materialien bevorzugt selbst nicht zulange Wärme, sodass diese Materialien sich relativ schnell aufheizen und wieder abkühlen. Dies erlaubt eine erhöhte Sicherheit, da nach der Behandlungsphase sichergestellt werden kann, dass die Behandlungsfläche aufgrund von Restwärme keine Gefährdung darstellt. Darüber hinaus zeichnen sich sowohl Keramik als auch Gold durch eine hohe biologische Verträglichkeit bei den bevorzugten Behandlungstemperaturen aus. Allergische Reaktion oder anderen unerwünschte Nebenwirkungen können bei dieser Wahl der Materialien besonders effektiv vermieden werden.

In einer bevorzugten Ausführungsform der Erfindung ist die Vorrichtung dadurch gekennzeichnet, dass die Behandlungsfläche durch eine Markierung umrandet ist, welcher in Abhängigkeit des Behandlungszyklus leuchtet. Als Markierung kann es zum Beispiel bevorzugt sein die Behandlungsfläche mit einem Lichtleiter zu umranden. Dieser kann beispielsweise während der Aufheizphase oder aber während der Behandlungsphase zum Leuchten gebracht werden. Es hat sich gezeigt, dass durch eine explizite leuchtende Anzeige der Position der Behandlungsfläche der Erfolg der hyperthermischen Behandlung erhöht werden kann. So wird durch die visuelle Markierung ein zentriertes Auflegen auf den betroffenen Hautpartien gefördert, sodass der Wärmeimpuls zielgerichtet auf diese Hautpartien aufgebracht werden kann. Durch die beleuchtete Markierung kann eine Benutzung auch im Dunkeln, beispielsweise in einem Zelt im Freien bei Nacht, unproblematisch vorgenommen werden kann.

In einer weiteren bevorzugten Ausführungsform der Erfindung ist die Vorrichtung dadurch gekennzeichnet, dass die Vorrichtung eine optische Anzeige und/oder einen Schallgeber umfasst, welcher den Start der Aufheizphase, das Erreichen der Behandlungstemperatur, die Dauer der Behandlungsphase und/oder das Ende der Behandlungsphase durch ein akustisches und/oder optisches Signal anzeigt. Die optische Anzeige kann bevorzugt durch Leuchtdioden (LED), Glühlampen, Flüssigkeitskristall (LCD) Displays oder andere bekannte optischen Anzeigen erfolgen. Bevorzugt kommt ein Farbcode zum Tragen, welcher an die Funktion angepasst ist. Beispielsweise kann die Aufheizphase durch ein oranges Signal angezeigt werden, die Behandlungsphase durch ein rotes Signal und das Ende der Behandlungsphase durch ein grünes Signal. Die akustische Signalgebung erfolgt bevorzugt durch einen Lautsprecher, welcher besonders bevorzugt kurze oder längere Pieptöne aussendet. Durch die optische und/oder akustische Signalgebung erfährt der Nutzer zu jeder Zeit der Vorbereitungs- oder Behandlungsphase den Zustand der Vorrichtung. Überraschenderweise ergibt sich dadurch eine zusätzlich psychologische Wirkung, welche durch eine Konzentration auf die Signalgebung zu einer noch stärkeren Minderung des Juckreizes führt. Weiterhin wird durch die bevorzugte Ausführungsform die Bedienungsfreundlichkeit und -sicherheit sowie die Compliance der Patienten in großem Maße gesteigert. Zudem erlaubt die optische und/oder akustische Signalgebung weitere Sicherheitsmechanismen einzuführen. So kann das Überschreiten einer Maximaltemperatur dem Nutzer schnell und deutlich angezeigt werden. Hierdurch kann der Benutzer die Behandlungsfläche von der Hautpartie entfernen, bevor Hautschädigungen auftreten können. Es hat sich gezeigt, dass die Reaktion durch Schmerzempfindungen zum Eigenschutz deutlich langsamer ist, als die Reaktion auf ein optisches und/oder akustisches Warnsignal, sodass die Signalgebung ein zusätzliches effektives Sicherheitsmerkmal der Vorrichtung darstellt.

In einer weiteren bevorzugten Ausführungsform umfasst die Vorrichtung ein wasserdichtes Gehäuse. Das Gehäuse stellt bevorzugt eine äußere Ummantelung der Vorrichtung dar, sodass diese insbesondere die Regelvorrichtung und anderen elektronische Komponenten umschließt. Es ist bevorzugt, dass das Gehäuse einen Gehäusekopf und einen Gehäusegriff aufweist, wobei die Behandlungsfläche bevorzugt an einem unteren Abschnitt des Gehäusekopfes vorliegt. Zur Steuerung und Temperierung der Behandlungsfläche weist das Gehäuse bevorzugt an der entsprechenden Position einen Durchbruch auf. In der bevorzugten Ausführungsform ist das Gehäuse derart ausgestaltet, dass sämtliche Durchbrüche, d.h. zum Beispiel ebenfalls eventuell vorhandene Batteriefächer, wasserdicht sind. Beispielsweise können zu diesem Zweck Dichtungsringe oder geeignete Dichtungen, zum Beispiel aus Elastomeren, eingesetzt werden. Der Fachmann kennt jedoch viele weitere technische Möglichkeiten, um ein Gehäuse wasserdicht zu konstruieren. Die wasserdichte Ausgestaltung des Gehäuses stellt ein zusätzliches Sicherheitselement dar, da hierdurch Schäden an der Regelvorrichtung oder anderen elektronische Komponenten aufgrund von eintretenden Flüssigkeiten wirksam vermieden werden können. Auch führt das wasserdichte Gehäuse zur Vermeidung von Korrosion und somit zu einer verlängerten Lebensdauer der Vorrichtung.

Besonders ist die Erfindung in dieser bevorzugten Ausführungsform in Kombination mit der beleuchteten Markierung auch für eine Benutzung unter besonderen Bedingungen, beispielsweise bei Expeditionen in zivilisationsferne Gegenden, bei unter Umständen feuchtheißem Klima, geeignet.

In einer weiteren bevorzugten Ausführungsform der Erfindung ist die Vorrichtung dadurch gekennzeichnet, dass die Vorrichtung eine Stromversorgungseinheit umfasst sowie einen Spannungswächter, welcher die Spannung der Stromversorgungseinheit überwacht. Im Sinne der Erfindung stellt die Stromversorgungseinheit bevorzugt die elektrische Energie zum Betreiben der Vorrichtung bereit. Bevorzugte Stromversorgungseinheiten sind Batterien oder Akkus. Diese stellen die elektrische Energie zumeist durch Bereitstellung einer Gleichspannung zur Verfügung. In der bevorzugten Ausführungsform wird die durch die Stromversorgungseinheit bereitgestellte Spannung mit Hilfe eines Spannungswächters überwacht. Im Sinne der Erfindung bezeichnet ein Spannungswächter bevorzugt eine elektrische Schaltung, welche die Spannung der Stromversorgungseinheit messen kann und eine Aktion auslöst, wenn diese unter einen vorgegebenen Grenzwert fällt. Im Stand der Technik sind eine Vielzahl von Varianten für Spannungswächter bekannt, wobei der Fachmann weiß, welcher Spannungswächter für welche Arten von Stromversorgungseinheiten, d.h. insbesondere Batterien oder Akkus, geeignet ist. Es ist bevorzugt, dass falls der Spannungswächter einen Abfall der Spannung der Stromversorgungseinheit unter einen bestimmten Wert feststellt, dieser eine Unterbrechungsaufforderung (engl. *interrupt request, IRQ*) an die Regelvorrichtung sendet, welche bevorzugt ein Mikroprozessor ist. Falls währenddessen ein Behandlungszyklus, d.h. ein Aufheizen oder eine Behandlungsphase, durchlaufen wird, führt der *interrupt request* zu einem Abbruch des Behandlungszyklus. Dies stellt einen weiteren Sicherheitsmechanismus dar. So wurde festgestellt, dass eine Unterspannung an der Stromversorgungseinheit zu einem Ausfall der Regelvorrichtung, z.B. des Mikroprozessor führen kann. In dem Fall kann es dazu kommen, dass die Temperaturregulation der Behandlungstemperatur mit Hilfe der Regelvorrichtung fehlerhaft ausgeführt wird und es zu einem unkontrollierten Erhitzen der Behandlungsfläche kommt. Der Spannungswächter kann somit zusätzlich dazu beitragen die Sicherheit der Vorrichtung zu erhöhen und eine Gesundheitsgefährdung im Falle beispielsweise einer fehlerhaften Batterie zu vermeiden.

In einer bevorzugten Ausführungsform ist die Vorrichtung dadurch gekennzeichnet, dass die Regelvorrichtung einen Mikroprozessor umfasst. Im Sinne der Erfindung wird unter einem Mikroprozessor bevorzugt eine Datenverarbeitungsvorrichtung, d.h. ein Prozessor, verstanden, welcher sich durch kleine Abmaße im Bereich von einigen mm auszeichnet und wobei bevorzugt alle Bausteine des Prozessors auf einem Mikrochip oder auch integriertem Schaltkreis (engl. *integrated circuit*, IC). Der Mikroprozessor kann bevorzugt auch ein Mikrokontroller sein, welcher neben dem Prozessor weitere periphere Elemente auf dem mikrochip integriert und beispielsweise auch über einen Datenspeicher verfügt. Es ist weiterhin bevorzugt, dass der Mikroprozessor auf einer Leiterplatte (engl. *printed circuit board*, PCB) installiert vorliegt. Außer dem Mikroprozessor liegen auf dem PCB weiterhin bevorzugt die Heizelemente sowie die Temperatorsensoren installiert vor. Diese bevorzugte Ausführungsform erlaubt eine äußert kompakte und ebenso robuste Bauweise der Vorrichtung und eine besonders intelligente Temperaturregulation mit Hilfe des Mikroprozessors. So ist der Mikroprozessor nicht nur in der Lage die gemessenen Temperaturdaten auszuwerten und in einer Steuerung der Heizelemente zu übersetzen, sondern kann weiterhin andere Parameter wie Fehlermeldungen und Benutzerinput schnell und zuverlässig berücksichtigen.

In einer bevorzugten Ausführungsform der Erfindung ist die Vorrichtung dadurch gekennzeichnet, dass der Mikroprozessor, das Heizelement und der mindestens eine Temperatursensor auf einem *printed circuit board* (PCB) installiert sind, wobei mindestens das Heizelement und der Temperatursensor mit Hilfe eines Schutzlackes beschichtet sind. Im Sinne der Erfindung wird unter dem Schutzlack bevorzugt ein Lack oder eine Farbe verstanden, welcher Bauteile des PCBs vor Umwelteinflüssen schützen soll. Der Schutzlack wirkt zu diesem Zweck bevorzugt elektrisch isolierend und ist wasserbeständig. Die Eigenschaft der elektrischen Isolation kann bevorzugt anhand des Oberflächenwiderstandes oder engl. *surface insulation resistance* (SIR) quantifiziert werden. Der SIR kann bevorzugt beispielsweise durch Leckströme zwischen den Bauteilen der Leiterplatte gemessen werden. Ein hoher Widerstand entspricht einer guten elektrischen Isolation. Wasserbeständig meint bevorzugt, dass auch bei einer hohen Luftfeuchtigkeit oder dem Eindringen von Wasser die lackierten elektronischen Bauteile intakt bleiben und es zu keinem Kurzschluss kommt. Die Wasserbeständigkeit kann beispielsweise ebenfalls unter Messung der SIR unter Bedingungen mit hoher Luftfeuchtigkeit getestet werden. Im Stand der Technik ist eine Vielzahl von Schutzlacken bekannt, welche bevorzugt verwandt werden können. Beispielhaft seien Schutzlacke auf Acryl-, Silikon- oder Polyurethanbasis genannt. Durch die Applikation des Schutzlackes im Bereich der Heizelemente und Temperatursensoren werden diese vor Ablagerungen wirksam geschützt, sodass Fehlmessungen der Temperatursensoren vermieden werden können. Dies erhöht zum einen die Genauigkeit mit welcher die Behandlungstemperatur eingestellt werden kann und vermeidet zum anderen, dass aufgrund einer Fehlmessung der Temperatur es zu einem Überhitzen der Behandlungsfläche kommt.

Die Beschichtung der genannten Bauelemente erlaubt auf überraschende Weise eine zuverlässige, zusätzliche Temperatursicherung der Vorrichtung mit besonders einfachen und kostengünstigen technischen Mitteln. Besonders vorteilhaft für die Beschichtung der Bauelemente hat sich überraschenderweise eine Installation der Bauteile auf einem PCB erwiesen.

In einer bevorzugten Ausführungsform der Erfindung ist die Vorrichtung dadurch gekennzeichnet, dass die Vorrichtung einen Datenspeicher zur Speicherung der Systemdaten und/oder Fehlermeldungen umfasst. Zu den bevorzugten Systemdaten gehört ein Zähler für die Behandlungszyklen, welcher bevorzugt separat die Verwendung verschiedene Typen von Behandlungszyklen zählt. Kann beispielsweise ein kurzer oder ein langer Behandlungszyklus ausgewählt werden, so wird diese separate gezählt. Weiterhin umfassen die Systemdaten bevorzugt einen Boot Zähler, also eine Zähler dafür wie oft die Vorrichtung gestartet wurde sowie eine Angabe der Fehlermeldungen mit aktuellem Fehlerstand.

Bevorzugt können folgende Fehlermeldungen gespeichert: Ein "Reset" zeigt an, dass der Spannungswächter einen Reset ausgelöst hat. Ein "Watchdog" zeigt an, dass in der Firmware ein Watchdog-Reset aufgetreten ist, d.h. ein Systemneustart aufgrund eines Softwarefehlers. Bevorzugt kann für die Fehlermeldung festgestellt werden in welchem Programmmodus sich das Gerät bei dem Auftreten des Fehlers befand. Ein "Temperatur zu hoch" kann anzeigen, dass die am Temperatursensor gemessene Temperatur zu hoch ist, oder dass der Temperatursensor defekt ist. Ein "Temperatur zu niedrig" kann anzeigen, dass die am Temperatursensor gemessene Temperatur zu niedrig ist, oder dass der Temperatursensor defekt ist. Ein "Behandlungstemperatur erreicht" kann anzeigen, ob die gewünschte Behandlungstemperatur erreicht wurde oder ein Fehler während der Vorheizphase aufgetreten ist.

Vorteilhafterweise können die gespeicherten Systemdaten und Fehlermeldungen für die Diagnose und Problembehandlung für die Vorrichtung eingesetzt werden. So können diese Daten beispielsweise ausgelesen werden, wenn ein Kunde ein defektes Gerät einsendet. Anhand der Daten ist es möglich den aufgetretenen Fehler, z.B. "Temperatur zu hoch", mit weiteren Systemdaten zur Anzahl der Behandlungszyklen oder Watchdog-Resets zu korrelieren. Anhand dieser Daten kann somit sowohl während der Entwicklungsphase, als auch danach, die Sicherheitsmerkmale der Vorrichtung kontinuierlich optimiert werden. Die Möglichkeit, dass die Vorrichtung eine Speicherung von Systemdaten und Fehlermeldungen umfasst, erlaubt somit die kontinuierliche Verbesserung der Hardwaresowie Softwarebestandteile der Vorrichtung anhand aussagekräftiger Daten.

In einer weiteren bevorzugten Ausführungsform ist die Vorrichtung dadurch gekennzeichnet, dass auf der Regelvorrichtung einer Firmware installiert vorliegt, welche mindestens die Temperaturregulation der Behandlungsfläche steuert, wobei die Firmware einen watchdog counter (WDC) umfasst, welcher überwacht, ob die Firmware ausgeführt wird. Im Sinne der Erfindung wird bevorzugt unter der Firmware eine Software, d.h. die Anleitung für ein computer-implementiertes Verfahren verstanden, welches in der Regelvorrichtung bevorzugt in dem Mikroprozessor eingebettet vorliegt. D.h. die Firmware umfasst bevorzugt jene Software, welche mit der Hardware der Vorrichtung, d.h. insbesondere mit den Heizelementen und Temperatursensoren funktional verbunden ist. Vorzugsweise wird die Firmware mit dem Start der Vorrichtung ausgeführt und übernimmt die Kontroll- und Steuerungsfunktion dieser Hardware-Komponenten der Vorrichtung. Somit wertet die Regelvorrichtung bevorzugt auf Basis der Firmware z.B. die Messdaten der Temperatursensoren sowie Nutzereingaben aus, um während des Behandlungszyklus die Stromzufuhr für die Heizelemente zu steuern. Im Sinne der Erfindung bezeichnen hardwareimplementierte Bauelemente bevorzugt Bauelemente, deren Funktion unabhängig von einer korrekten Ausführung der Firmware sichergestellt ist. Wie obig beschrieben ist der Temperaturwächter hardwareimplementiert, sodass dessen Funktion, d.h. eine Begrenzung der Maximaltemperatur, unabhängig von einer korrekten Ausführung der Firmware auf der Regelvorrichtung erfolgen kann. Selbst bei einem Systemabsturz der Firmware kann der hardwareimplementierte Temperaturwächter daher schnell und korrekt die Maximaltemperatur der Behandlungsfläche begrenzen.

In der besonders bevorzugten Ausführungsform wird die Firmware der Regelvorrichtung mit Hilfe eines hardwareimplementierten Watchdog Counters überwacht. Besonders bevorzugt handelt es sich dabei um einen Time-out-Watchdog. Bevorzugt wird der Time-Out-Watchdog vor dem Start der Behandlungsphase durch die Firmware aktiviert. Während der Behandlungsphase wird von der Firmware innerhalb eines vorbestimmten Zeitintervalls ein Signal an den Time-Out-Watchdog gesendet, um diesen zurückzusetzen. Falls der Time-Out-Watchdog nicht zurückgesetzt wird, führt dies bevorzugt zu einem Neustart der Firmware. Das Zeitintervall richtet sich bevorzugt an die Zeit, welche vorgesehen ist um eine Temperaturmessung und Regelung der Heizelemente durch die Firmware durchzuführen und kann z.B. zwischen 2 ms und 10 ms betragen. Durch einen solchen Time-Out-Watchdog kann vorteilhafterweise sichergestellt werden, dass mindestens während der Behandlungsphase der Vorrichtung die Firmware korrekt funktioniert und die Temperatur der Behandlungsfläche überwacht wird. Durch einen hardwareimplementierten Watchdog zur Überwachung der Firmware, bevorzugt zum Beispiel mit Hilfe eines Time-Out-Watchdogs, kann somit sichergestellt werden, dass falls die Firmware nicht korrekt funktioniert und das vorgegebene Zeitintervall nicht eingehalten wird, die Behandlungsphase abgebrochen wird. Somit ist ein weiteres Sicherheitsfeature der Vorrichtung zusätzlich zu den oben erwähnten vorhanden, welches insbesondere im Zusammenspiel mit dem hardwareimplementierten Temperaturwächter dafür sorgt, dass auch bei nicht korrekt funktionierender Firmware ein Überhitzen der Behandlungsfläche ausgeschlossen ist. Als letzte, zusätzliche Sicherheitsstufe ist dann ergänzend die Schmelzsicherung vorhanden.

Dies stellt sicher, dass die Behandlung stets mit den optimierten Parametern für die hyperthermische Behandlung ausgeführt wird, und kann als weitere Sicherheitsschranke die Behandlungsfläche vor einem Überhitzen schützen.

### Beschreibung der Figuren

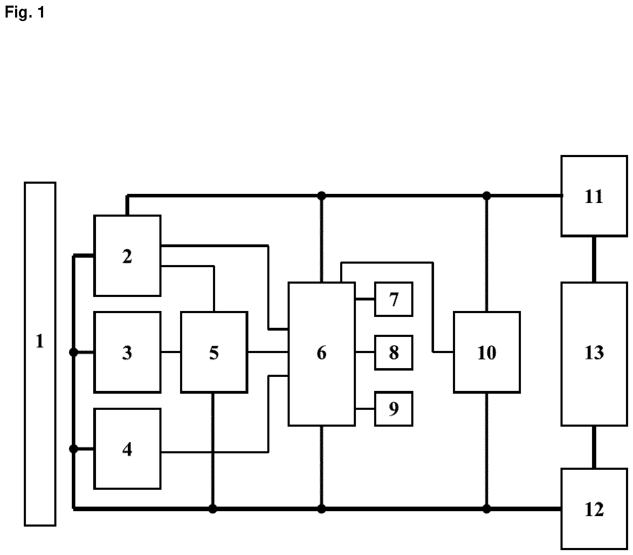

- Fig. 1: Blockschaltbild einer bevorzugten Ausführungsform der Vorrichtung

Fig. 1 zeigt das Blockschaltbild einer bevorzugten Ausführungsform der Vorrichtung. Die Behandlungsfläche 1 wird mit Hilfe von mindestens einem Heizelement 2 auf die Behandlungstemperatur erhitzt. Bevorzugt handelt es sich bei der Behandlungsfläche 1 um ein Kontaktpad, welches aus Keramik oder Gold gefertigt ist. Als Heizelemente 2 haben sich insbesondere Halbleiter-Bauelemente wie FETs, ganz bevorzugt MOSFETs, als vorteilhaft erwiesen. Neben der kleinen Dimensionierung zeichnen sich diese durch eine sehr dynamische Wärmeerzeugung und -abgabe und ein besonders schnelles Ansprechverhalten aus. Die Regulation der Temperatur der Behandlungsfläche 1 erfolgt mit Hilfe einer Regelvorrichtung 6, welche bevorzugt ein Mikrokontroller ist, und die Stromzufuhr zu den Heizelementen 2 steuert. Dabei erfolgt eine Überwachung der Temperatur der Behandlungsfläche 1 mit Hilfe eines ersten Temperatursensors 4, welcher bevorzugt ein NTC-Thermotransistor ist. Aufgrund der gemessenen Temperatur durch den Temperatursensor 4 kann die Regelvorrichtung 6 die optimale Stromzufuhr einstellen, um die Behandlungstemperatur in dem gewünschten Zeitraum konstant zu halten. Während des Betriebes der Vorrichtung zeigt eine visuelle Anzeige 7, bevorzugt eine LED, sowie ein Schallgeber 8, bevorzugt ein Buzzer, dem Benutzer die Phase des Behandlungszyklus an. So kann beispielsweise beim Start der Aufheizphase der Schallgeber 8 einen Buzzton genieren und die optische Anzeige 7 zum Aufleuchten gebracht werden. Die Eingabe der bevorzugten Behandlungszeit erfolgt durch das Eingabeelement 9, welches bevorzugt ein Taster ist. Die verschiedenen Programmabläufe sowie die Regulation der Temperatur der Behandlungsfläche 1 werden durch die Regelvorrichtung 6 gesteuert. Die Strom- bzw. Spannungsversorgung der Vorrichtung wird durch eine Stromversorgungseinheit 13, z.B. eine Batterie, bereitgestellt.

Falls die Regelvorrichtung 6 zur Steuerung der Temperatur der Behandlungsfläche 1 aufgrund z.B. einer fehlerhaften Elektronik versagt, erlaubt der hardwareimplementierte Temperaturwächter ein Abschalten der Heizelemente 2 bei Überschreiten einer Maximaltemperatur. Zu diesem Zweck weist die Vorrichtung einen zweiten Temperatursensor 3, bevorzugt einen NTC-Thermistor, auf, welcher mit einem Komparator 5 verbunden ist. Steigt die Temperatur der Behandlungsfläche 1, bewegt sich der durch den zweiten Temperatursensor 3 gesteuerte Spannungswert am Komparator 5 auf einen Referenzwert der Spannung zu, welcher einer vorher festgelegten Maximaltemperatur entspricht. Sobald die Temperatur der Behandlungsfläche 1 die Maximaltemperatur überschreitet, ändert sich das Ausgangsignal am Komparator 5 binär, wodurch die Versorgungsspannung der Heizelemente 2 unterbrochen wird.

Neben dem hardwareimplementierten Temperaturwächter weist die Vorrichtung zudem eine Schmelzsicherung 11 auf, welche als ein zusätzliches Sicherungselement fungiert. Während der Behandlungsphase wird von den Heizelementen 2 in einem störungsfreien Betrieb ein definierter Behandlungsstrom von der Stromversorgungseinheit 13 bezogen. Kommt es aufgrund eines Defektes zu einem Durchheizen der Heizelemente 2 steigt der Versorgungsstrom stark an. Vorteilhafterweise wird ab einem maximalen Strom die Schmelzsicherung 11 ausgelöst. Hierdurch kann selbst bei einem Kurzschluss der Vorrichtung ein unkontrolliertes Aufheizen der Behandlungsfläche 1 vermieden werden. Vorteilhafterweise muss dieser Sicherungsmechanismus nur eingreifen, falls sowohl die Temperaturregulation durch die Regelvorrichtung 6 als auch durch den hardwareimplementierten Temperaturwächter nicht ordnungsgemäß funktioniert. Zur weiteren Kontrolle der Stromversorgungseinheit 13 umfasst die Vorrichtung zudem einen Spannungsversorgungs-Verpolschutz 12 sowie einen Spannungswächter 10, welcher bevorzugt ein *supply voltage supervisor* (SVS) ist.

### Bezugszeichenliste

- 1: Behandlungsfläche
- 2: Heizelement(e)
- 3: zweiter Temperatursensor für den hardwareimplementierten Temperaturwächter
- 4: erster Temperatursensor für die Steuerung durch die Regelvorrichtung
- 5: Komparator des hardwareimplementierten Temperaturwächters
- 6.: Regelvorrichtung
- 7: optische Anzeige
- 8: Schallgeber
- 9: Eingabeelement für die Behandlungszeit
- 10: Spannungswächter
- 11: Schmelzsicherung
- 12: Spannungsversorgung-Verpolschutz
- 13: Stromversorgungseinheit

## Patentansprüche

1. Vorrichtung zur hyperthermischen Behandlung von Juckreiz umfassend
a) mindestens eine Behandlungsfläche (1)
und
b) eine Regelvorrichtung (6) zur Regulation der Temperatur der Behandlungsfläche (1), wobei
die Regelvorrichtung (6) die Behandlungsfläche (1) durch Erhitzen mindestens eines Heizelementes (2) in einer Aufheizphase auf eine Behandlungstemperatur zwischen 42 °C und 56 °C regulieren kann und die Behandlungstemperatur in einer Behandlungsphase für einen Zeitraum von 2 sec bis 12 sec gehalten werden kann,
**dadurch gekennzeichnet, dass**
zusätzlich ein hardwareimplementierter Temperaturwächter die Maximaltemperatur der Behandlungsfläche (1) auf einen Wert innerhalb einer Toleranzspanne um 56°C von weniger als +-10% reversibel begrenzt
und
eine Schmelzsicherung (11) bei Kurzschluss oder ungeregeltem Durchheizen die Vorrichtung abschaltet.

2. Vorrichtung gemäß dem vorherigen Anspruch
**dadurch gekennzeichnet, dass**
die Vorrichtung mindestens einen ersten Temperatursensor (4) zur Messung der Temperatur der Behandlungsfläche (1) umfasst und die Regelvorrichtung (6) basierend auf den Messdaten des Temperatursensors (4) die Temperatur des mindestens einen Heizelementes (2) einstellt.

3. Vorrichtung gemäß dem vorherigen Anspruch
**dadurch gekennzeichnet, dass**
der hardwareimplementierte Temperaturwächter mindestens einen zweiten Temperatursensor (3) zur Messung der Temperatur der Behandlungsfläche (1) und einen Komparator (5) umfasst, wobei der Komparator (5) die Temperatur der Behandlungsfläche (1) mit der Maximaltemperatur vergleicht und bei Überschreiten der Maximaltemperatur die Stromzufuhr zu dem Heizelement (2) unterbindet.

4. Vorrichtung gemäß einem der vorherigen Ansprüche
**dadurch gekennzeichnet, dass**
die Schmelzsicherung (11) einen Schwellenwert für einen maximalen Strom aufweist, welcher der Erhitzung der Behandlungsfläche (1) auf 65 °C für 1 Sekunde entspricht.

5. Vorrichtung gemäß dem vorherigen Anspruch
**dadurch gekennzeichnet, dass**
der Schwellenwert der Schmelzsicherung (11) bevorzugt zwischen 1 A und 2,5 A besonders bevorzugt ungefähr 2 A beträgt.

6. Vorrichtung gemäß einem der vorherigen Ansprüche
**dadurch gekennzeichnet, dass**
die Behandlungsfläche (1) Keramik und/oder Gold umfasst.

7. Vorrichtung gemäß einem der vorherigen Ansprüche
**dadurch gekennzeichnet, dass**
die Behandlungsfläche (1) durch eine Markierung umrandet ist, welche während der Behandlungsphase leuchtet.

8. Vorrichtung gemäß einem der vorherigen Ansprüche
**dadurch gekennzeichnet, dass**
die Vorrichtung eine optische Anzeige (7) und/oder einen Schallgeber (8) umfasst, welcher den Start der Aufheizphase, das Erreichen der Behandlungstemperatur, die Dauer der Behandlungsphase und/oder das Ende der Behandlungsphase durch ein akustisches und/oder optisches Signal anzeigt.

9. Vorrichtung gemäß einem der vorherigen Ansprüche
**dadurch gekennzeichnet, dass**
die Vorrichtung ein wasserdichtes Gehäuse umfasst.

10. Vorrichtung gemäß einem der vorherigen Ansprüche
**dadurch gekennzeichnet, dass**
die Vorrichtung eine Stromversorgungseinheit (13) umfasst sowie einen Spannungswächter (10), welcher die Spannung der Stromversorgungseinheit (13) überwacht.

11. Vorrichtung gemäß einem der vorherigen Ansprüche
**dadurch gekennzeichnet, dass**
die Regelvorrichtung (6) mindestens einen Mikroprozessor umfasst.

12. Vorrichtung gemäß dem vorherigen Anspruch
**dadurch gekennzeichnet, dass**
der Mikroprozessor, das mindestens eine Heizelement (2) und mindestens ein Temperatursensor (3, 4) auf einem *printed circuit board* (PCB) installiert vorliegen, wobei mindestens das Heizelement (2) und der Temperatursensor (3, 4) mit Hilfe eines Schutzlackes beschichtet sind.

13. Vorrichtung gemäß einem der vorherigen Ansprüche
**dadurch gekennzeichnet, dass**
die Vorrichtung einen Datenspeicher zur Speicherung von Systemdaten und/oder Fehlermeldungen umfasst.

14. Vorrichtung gemäß einem der vorherigen Ansprüche
**dadurch gekennzeichnet, dass**
auf der Regelvorrichtung (6) eine Firmware installiert vorliegt, welche bei der Inbetriebnahme der Vorrichtung gestartet wird und mindestens die Temperaturregulation der Behandlungsfläche (1) steuert, wobei die Regelvorrichtung (6) einen hardwareimplementierten Watchdog Counter (WDC) umfasst, welcher überwacht, ob die Firmware ausgeführt wird.

## Claims

1. A device for hyperthermal treatment of itching, comprising
a) at least one treatment surface (1)
and
b) a control device (6) for regulating the temperature of the treatment surface (1), wherein
the control device (6) can regulate the treatment surface (1) by heating at least one heating element (2) in a heating phase to a treatment temperature between 42°C and 56°C, and the treatment temperature can be maintained in a treatment phase for a period of 2 sec to 12 sec,
**characterized in that**
in addition, a hardware-implemented temperature monitor reversibly limits the maximum temperature of the treatment surface (1) to a value within a tolerance range around 56°C of less than +-10%
and
a fuse (11) switches off the device in the event of a short circuit or uncontrolled continuous heating.

2. The device according to the preceding claim,
**characterized in that**
the device comprises at least one first temperature sensor (4) for measuring the temperature of the treatment surface (1) and the control device (6) adjusts the temperature of the at least one heating element (2) based on the measurement data of the temperature sensor (4).

3. The device according to the preceding claim,
**characterized in that**
the hardware-implemented temperature monitor comprises at least a second temperature sensor (3) for measuring the temperature of the treatment surface (1) and a comparator (5), wherein the comparator (5) compares the temperature of the treatment surface (1) to the maximum temperature and interrupts the power supply to the heating element (2) if the maximum temperature is exceeded.

4. The device according any one of the preceding claims,
**characterized in that**
the fuse (11) has a threshold value for a maximum current, which corresponds to heating the treatment surface (1) to 65°C for 1 second.

5. The device according to the preceding claim,
**characterized in that**
the threshold value of the fuse (11) is preferably between 1 A and 2.5 A, particularly preferably about 2 A.

6. The device according any one of the preceding claims,
**characterized in that**
the treatment surface (1) comprises ceramic and/or gold.

7. The device according any one of the preceding claims,
**characterized in that**
the treatment area (1) is bordered by a marking which lights up during the treatment phase.

8. The device according any one of the preceding claims,
**characterized in that**
the device comprises a visual indicator (7) and/or a sound generator (8), which indicates the start of the heating phase, the reaching of the treatment temperature, the duration of the treatment phase, and/or the end of the treatment phase by means of an acoustic and/or visual signal.

9. The device according any one of the preceding claims,
**characterized in that**
the device comprises a waterproof housing.

10. The device according any one of the preceding claims,
**characterized in that**
the device comprises a power supply unit (13) and a voltage monitor (10), which monitors the voltage of the power supply unit (13).

11. The device according any one of the preceding claims,
**characterized in that**
the control device (6) comprises at least one microprocessor.

12. The device according to the preceding claim,
**characterized in that**
the microprocessor, the at least one heating element (2), and at least one temperature sensor (3, 4) are installed on a *printed circuit board* (PCB), wherein at least the heating element (2) and the temperature sensor (3, 4) are coated by means of a protective coating.

13. The device according any one of the preceding claims,
**characterized in that**
the device comprises a data memory for storing system data and/or error messages.

14. The device according any one of the preceding claims,
**characterized in that**
a firmware is installed on the control device (6), which firmware is started when the device is put into operation and controls at least the temperature regulation of the treatment surface (1), wherein the control device (6) comprises a hardware-implemented watchdog counter (WDC), which monitors whether the firmware is executed.

## Revendications

1. Dispositif pour le traitement hyperthermique de démangeaisons comprenant
a) au moins une surface de traitement (1)
et
b) un dispositif de régulation (6) pour la régulation de la température de la surface de traitement (1),
dans lequel
le dispositif de régulation (6) peut réguler la surface de traitement (1) par chauffage d'au moins un élément chauffant (2) dans une phase de chauffage à une température de traitement comprise entre 42 °C et 56 °C et la température de traitement peut être maintenue dans une phase de traitement pendant une période de 2 sec à 12 sec,
**caractérisé en ce que**
un contrôleur de température à implémentation matérielle limite en plus de manière réversible la température maximale de la surface de traitement (1) à une valeur à l'intérieur d'une marge de tolérance de 56 °C inférieure à ±10 %
et
un fusible (11) met le dispositif hors service en cas de court-circuit ou de chauffage continu non régulé.

2. Dispositif selon la revendication précédente,
**caractérisé en ce que**
le dispositif comprend au moins un premier capteur de température (4) pour la mesure de la température de la surface de traitement (1) et le dispositif de régulation (6) sur la base des données de mesure du capteur de température (4) règle la température du au moins un élément chauffant (2).

3. Dispositif selon la revendication précédente,
**caractérisé en ce que**
le contrôleur de température à implémentation matérielle comprend au moins un deuxième capteur de température (3) pour la mesure de la température de la surface de traitement (1) et un comparateur (5), dans lequel le comparateur (5) compare la température de la surface de traitement (1) à la température maximale et lors d'un dépassement de la température maximale empêche l'amenée de courant vers l'élément chauffant (2).

4. Dispositif selon l'une quelconque des revendications précédentes,
**caractérisé en ce que**
le fusible (11) présente une valeur seuil pour un courant maximal, laquelle correspond au chauffage de la surface de traitement (1) à 65 °C pendant 1 seconde.

5. Dispositif selon la revendication précédente,
**caractérisé en ce que**
la valeur seuil du fusible (11) est comprise de préférence entre 1 A et 2,5 A de manière particulièrement préférée atteint approximativement 2 A.

6. Dispositif selon l'une quelconque des revendications précédentes,
**caractérisé en ce que**
la surface de traitement (1) comprend de la céramique et/ou de l'or.

7. Dispositif selon l'une quelconque des revendications précédentes,
**caractérisé en ce que**
la surface de traitement (1) est bordée par un marquage, lequel brille pendant la phase de traitement.

8. Dispositif selon l'une quelconque des revendications précédentes,
**caractérisé en ce que**
le dispositif comprend un affichage optique (7) et/ou un générateur de son (8), lequel indique le début de la phase de chauffage, l'atteinte de la température de traitement, la durée de la phase de traitement et/ou la fin de la phase de traitement par un signal acoustique et/ou optique.

9. Dispositif selon l'une quelconque des revendications précédentes,
**caractérisé en ce que**
le dispositif comprend un boîtier étanche à l'eau.

10. Dispositif selon l'une quelconque des revendications précédentes,
**caractérisé en ce que**
le dispositif comprend une unité d'alimentation en courant (13) ainsi qu'un contrôleur de tension (10), lequel contrôle la tension de l'unité d'alimentation en courant (13).

11. Dispositif selon l'une quelconque des revendications précédentes,
**caractérisé en ce que**
le dispositif de régulation (6) comprend au moins un microprocesseur.

12. Dispositif selon la revendication précédente,
**caractérisé en ce que**
le microprocesseur, le au moins un élément chauffant (2) et au moins un capteur de température (3, 4) sont présents de manière installée sur une carte de circuits imprimés (PCB), dans lequel au moins l'élément chauffant (2) et le capteur de température (3, 4) sont revêtus à l'aide d'un vernis protecteur.

13. Dispositif selon l'une quelconque des revendications précédentes,
**caractérisé en ce que**
le dispositif comprend un magasin de données pour le stockage de données système et/ou messages d'erreur.

14. Dispositif selon l'une quelconque des revendications précédentes,
**caractérisé en ce que**
un micrologiciel, lequel est démarré lors de la mise en service du dispositif et commande au moins la régulation de température de la surface de traitement (1), est présent de manière installée sur le dispositif de régulation (6), dans lequel le dispositif de régulation (6) comprend un chien de garde compteur (WDC) à implémentation matérielle, lequel contrôle si le micrologiciel est exécuté.
